# EUROPÄISCHE PATENTSCHRIFT

(11) **EP 0 558 449 B1**
(45) Veröffentlichungstag und Bekanntmachung des Hinweises auf die Patenterteilung: **06.09.2000**
(21) Anmeldenummer: 93810100.3
(22) Anmeldetag: 18.02.1993
(51) Int. Cl.: C07F 7/08, C07F 5/00, C07F 7/00, C07F 5/06, C07F 15/00, A61K 31/695, C07H 23/00, C07J 51/00, C09B 47/00, A61K 31/58, A61K 31/70

(54) **Chelatkomplexe und Verfahren zu ihrer Herstellung**
Complexes of chelating ligands and process for their preparation
Complexes avec des ligands chélateurs et procédé pour les préparer

(30) Priorität: 27.02.1992 CH 60092
(43) Veröffentlichungstag der Anmeldung: 01.09.1993
(73) Patentinhaber: Novartis AG, 4058 Basel (CH); Novartis-Erfindungen Verwaltungsgesellschaft m.b.H., 1235 Wien (AT)
(72) Erfinder: Capraro, Hans Georg, Dr., CH-4310 Rheinfelden (CH); Baumann, Marcus, Dr., CH-4053 Basel (CH)

(56) Entgegenhaltungen:
- EP-A- 0 457 196
- WO-A-91/18006
- WO-A-92/01753
- JOURNAL OF THE AMERICAN CHEMICAL SOCIETY Bd. 112, 1990, Seiten 8064 - 8070 RIHTER, B.D. ET AL.
- CHEMICAL ABSTRACTS, vol. 116, 1992, Columbus, Ohio, US; abstract no. 37092w, CUOMO, V. ET AL. Seite 351 ;
- INORGANIC CHEMISTRY Bd. 6, Nr. 6, 1967, Seiten 1116 - 1120 ESPOSITO, J.N. ET AL.
- CHEMICAL ABSTRACTS, vol. 112, 1990, Columbus, Ohio, US; abstract no. 108644h, ERA, S. ET AL. Seite 680 ;
- CHEMICAL ABSTRACTS, vol. 114, 1991, Columbus, Ohio, US; abstract no. 111977r, KOBAYASHI, S. ET AL. Seite 672 ;
- CHEMICAL ABSTRACTS, vol. 115, 1991, Columbus, Ohio, US; abstract no. 243124r, PERRY, J.W. ET AL. Seite 750 ;
- CHEMICAL ABSTRACTS, vol. 110, 1989, Columbus, Ohio, US; abstract no. 143681y, SEMENOV, S.G. ET AL. Seite 570 ;

## Beschreibung

Die Erfindung betrifft Chelatkomplexe aus Phthalocyaninverbindungen als Komplexbildner und bestimmten organischen Derivaten von Elementen der dritten und vierten Hauptgruppe des Periodensystems mit einer Ordnungszahl von und mit 31 bis und mit 50 sowie von Aluminium oder Ruthenium als Zentralatom, pharmazeutische Zusammensetzungen, enthaltend diese Komplexe, Verfahren zu ihrer Herstellung und ihre Verwendung als Arzneimittel, zur Blutreinigung ausserhalb des Organismus und zur Diagnostik.

Einige Phthalocyaninkomplexe und ihre potentielle Verwendbarkeit in der photodynamisehen Therapie sind bereits bekannt, z.B. aus WO-A-92/01753, B. D. Rihter et al., J. Am. Chem. Soc. 112 (1990), 8064-8070, V. Cuomo et al., Br. J. Cancer 64 (1991), 93-95 und EP-A-0457196. J.D. Spikes, Photochem. Photobiol. 43, 691 (1986) beschreibt die Verwendung von Zink-phthalocyanin in der photodynamischen Chemotherapie zur Behandlung von Tumoren. Beispielsweise wurde Zink-phthalocyanin als wässerige Suspension an Mäuse oder Ratten intraperitoneal verabreicht und ein an den Versuchstieren zuvor erzeugtes Karzinom mit energiereichem Licht, vorzugsweise mit gebündeltem sichtbarem Licht (LASER), bestrahlt. Alle diese Phthalocyaninderivate unterscheiden sich aber von den erfindungsgemässen Verbindungen der unten gezeigten Formel I zumindest hinsichtlich der Reste R₁ und R₂, insbesondere hinsichtlich des Restes R₇.

Für die photodynamische Chemotherapie ist die Verweildauer der Komplexe im Organismus von entscheidender Bedeutung, weil die Patienten während der Behandlung gegen Licht ganz allgemein hochempfindlich sind und insbesondere Sonneneinstrahlung meiden müssen. Aus diesem Grund ist es auch erforderlich, Verbindungen zu finden, die sich möglichst selektiv im Tumor anreichern und in gesunden Teilen des Organismus nur in möglichst geringer Konzentration vorkommen. Für die humantherapeutische Verwendung spielt auch die Applikationsart eine Rolle. Intraperitoneale Applikation ist wegen der Schmerzen beim Einstich in die Bauchhöhle und der hohen Anforderungen an die Geschicklichkeit des Arztes generell problematisch. Es besteht daher ein Bedürfnis nach neuen Komplexen, die hinsichtlich der Applikationsweise, der Selektivität für Tumoren und/oder der Verweildauer im Organismus gegenüber den bekannten Komplexen Vorteile bieten.

Die Erfindung betrifft insbesondere Chelatkomplexe der Formel I, 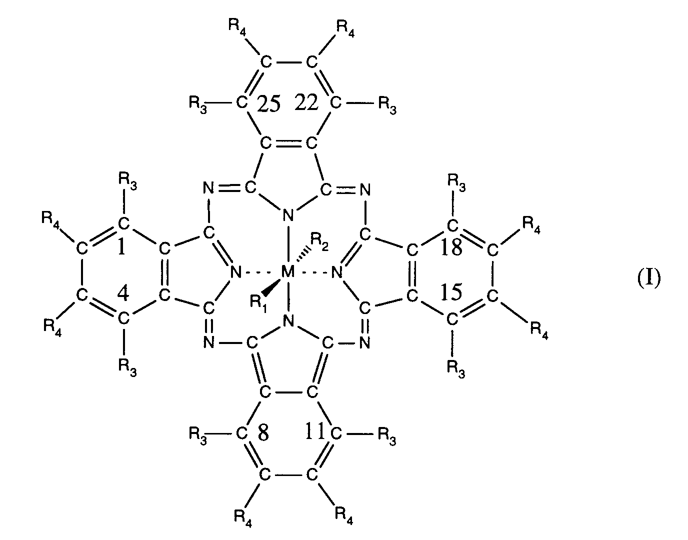 worin M ein Element der dritten oder vierten Hauptgruppe des Periodensystems mit einer Ordnungszahl von und mit 31 bis und mit 50, Aluminium oder Ruthenium bedeutet, R₁ für einen Rest der Formel II, 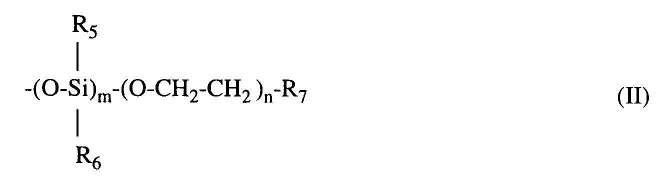 worin m für 0 oder 1 steht, n eine ganze Zahl von und mit 0 bis und mit 200, R₅ und R₆ unabhängig voneinander je Niederalkyl oder gegebenenfalls substituiertes Phenyl und R₇ Alkylthio mit bis zu 20 C-Atomen, Alkanoyloxy mit bis zu 20 C-Atomen, 4-(2,3,4,6-Tetra-O-acetyl-β-D-glucopyranos-1-yloxy)-phenoxy, Cholestan-3-yloxy, Cholesteryloxy, unsubstituiertes oder durch Cholestan-3-yl-oxy oder Cholesteryloxy substituiertes aliphatisches Hydrocarbyloxy mit bis zu 24 C-Atomen im aliphatischen Teil oder, wenn m für 1 und n für 0 steht, aliphatisches Hydrocarbyl mit 6-24 C-Atomen oder unsubstituiertes oder substituiertes Phenyl bedeuten, oder R₁ für einen Rest der Formel III steht, 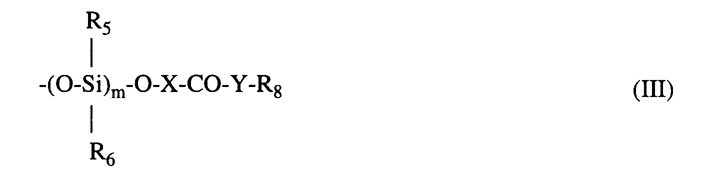 worin X einen bivalenten aliphatischen Kohlenwasserstoffrest mit bis zu 23 C-Atomen bedeutet, Y für Sauerstoff oder die Gruppe -NH- steht, R₈ für Cholesteryl, aliphatisches Hydrocarbyl mit bis zu 24 C-Atomen oder unsubstituiertes oder durch Cholesteryloxy substituiertes aliphatisches Hydrocarbyl mit bis zu 24 C-Atomen im aliphatischen Teil steht, und R₅, R₆ und m die obengenannten Bedeutungen haben, R₂ die Bedeutung von R₁ hat, wenn M ein Element der vierten Hauptgruppe ist, oder R₂ fehlt, wenn M ein Element der dritten Hauptgruppe oder Ruthenium ist, R₃ Wasserstoff, Niederalkyl, Niederalkylthio, unsubstituiertes oder durch Niederalkoxy substituiertes Alkoxy mit bis zu 20 C-Atomen, Triniederalkyl-silyl oder Halogen bedeutet und R₄ für Wasserstoff, Niederalkyl, Niederalkylthio, unsubstituiertes oder durch Niederalkoxy substituiertes Alkoxy mit bis zu 20 C-Atomen, Triniederalkyl-silyl oder Halogen steht, mit Ausnahme der Verbindung der Formel I, worin M für Zinn, R₁ und R₂ je für den Rest -OSi(C₆H₁₃)₃ und R₃ und R₄ je für tert. Butyl stehen, neue Zwischenprodukte zur Herstellung dieser Komplexe, pharmazeutische Zusammensetzungen, enthaltend diese Komplexe, Verfahren zu ihrer Herstellung und ihre Verwendung als Arzneimittel, zur Blutreinigung ausserhalb des Organismus und zur Diagnostik.

Ein Element der dritten oder vierten Hauptgruppe des Periodensystems mit einer Ordnungszahl von und mit 31 bis und mit 50 ist Gallium, Indium oder Zinn, sowie vorzugsweise Germanium.

In einem Rest der Formel II steht n vorzugsweise für eine ganze Zahl von und mit 0 bis und mit 20, hauptsächlich von und mit 0 bis und mit 5. Wenn n für eine Zahl grösser als 8 bis und mit 200 steht, so kann n auch die Durchschnittswerte in Verbindungsgemischen bedeuten.

Gegebenenfalls substituiertes Phenyl R₅ oder R₆ ist vorzugsweise unsubstituiertes Phenyl oder durch ein bis vorzugsweise nicht mehr als drei übliche Phenylsubstituenten, wie z.B. durch Niederalkyl, Niederalkoxy, Halogen, Trifluormethyl, Niederalkoxycarbonyl, Niederalkoxycarbonylamino, Benzoylamino und/oder Diniederalkylamino substituiertes Phenyl.

Niederalkyl R₅ und R₆ ist vorzugsweise Methyl oder n-Hexyl.

Alkylthio mit bis zu 20 C-Atomen R₇ hat vorzugsweise 10-18, hauptsächlich 10-14, C-Atome und ist ein solches verzweigtes oder insbesondere geradkettiges Alkylthio, wie vorzugsweise n-Dodecylthio.

Alkanoyloxy mit bis zu 20 C-Atomen R₇ ist vorzugsweise Niederalkanoyloxy, z.B. Acetoxy.

4-(2,3,4,6-Tetra-O-acetyl-β-D-glucopyranos-1-yloxy)-phenoxy R₇ ist der von Tetra-O-acetyl-arbutin abgeleitete Rest.

Cholestan-3-yl-oxy R₇ ist vorzugsweise 5α-Cholestan-3β-yloxy.

Aliphatisches Hydrocarbyl mit 6-24 C-Atomen R₇ ist ein aliphatischer Kohlenwasserstoffrest mit 6-24, insbesondere 6-20, C-Atomen, d.h. ein solcher Alkinyl- oder vorzugsweise Alkyl- oder Alkenylrest. Vorzugsweise hat ein solcher Hydrocarbylrest 12-18 C-Atome. Ein solcher Alkylrest ist z.B. geradkettig, wie n-Dodecyl, n-Tetradecyl, n-Octadecyl oder insbesondere n-Hexadecyl, oder verzweigt, wie 1,1,2-Trimethyl-propyl (Texyl). Ein solcher Alkenylrest hat vorzugsweise 18 C-Atome und ist z.B. cis- oder trans-9-Octadecenyl, -9,12-octadecadienyl oder -9,11,13-octadecatrienyl.

Unsubstituiertes oder substituiertes Phenyl R₇ ist vorzugsweise unsubstituiertes Phenyl. Substituiertes Phenyl R₇ ist insbesondere durch ein bis vorzugsweise nicht mehr als drei übliche Phenylsubstituenten, wie z.B. durch Niederalkyl, Niederalkoxy, Halogen, Trifluormethyl, Niederalkoxycarbonyl, Niederalkoxycarbonylamino, Benzoylamino und/oder Diniederalkylamino substituiertes Phenyl.

Aliphatisches Hydrocarbyloxy mit bis zu 24 C-Atomen R₇ ist über Sauerstoff gebundenes Hydrocarbyl mit bis zu 24, vorzugsweise bis zu 18 C-Atomen, d.h. ein solcher Alkinyloxy-oder vorzugsweise Alkoxy- oder Alkenyloxyrest Vorzugsweise hat ein solcher Hydrocarbyloxyrest 1-3 oder 12-18 C-Atome. Ein solcher Alkoxyrest ist z.B. geradkettig, wie n-Dodecyloxy, n-Tetradecyloxy, n-Octadecyloxy oder insbesondere n-Hexadecyloxy. Ein bevorzugter Alkoxyrest ist auch Methoxy. Ein solcher Alkenyloxyrest hat vorzugsweise 18 C-Atome und ist z.B. trans- oder Cis-9-Octadecenyloxy, -9,12-octadecadienyloxy oder -9,11,13-octadecatrienyloxy, vorzugsweise cis-9-Octadecenyloxy. Durch Cholesteryloxy oder Cholestan-3-yl-oxy substituiertes aliphatisches Hydrocarbyloxy R₇ ist vorzugsweise in ω-Stellung, d.h. endständig, durch Cholesteryloxy bzw. Cholestan-3-yl-oxy, wie insbesondere 5α-Cholestan-3β-yloxy, substituiertes aliphatisches Hydrocarbyloxy, wie insbesondere derartig substituiertes Alkoxy mit 12-18 C-Atomen, z.B. ω-Cholesteryloxyn-hexadecyloxy.

Aliphatisches Hydrocarbyl mit bis zu 24 C-Atomen R₈ ist ein aliphatischer Kohlenwasserstoffrest mit bis zu 24 C-Atomen, d.h. ein solcher Alkinyl- oder vorzugsweise Alkyl-oder Alkenylrest. Vorzugsweise hat ein solcher Hydrocarbylrest 12-18 C-Atome. Ein solcher Alkenylrest ist z.B. geradkettig, wie n-Dodecyl, n-Tetradecyl, n-Octadecyl oder insbesondere n-Hexadecyl, oder verzweigt, wie 1,1,2-Trimethyl-propyl (Texyl). Ein solcher Alkenylrest hat vorzugsweise 18 C-Atome und ist z.B. Cis- oder trans-9-Octadecenyl, - 9,12-octadecadienyl oder -9,11,13-octadecatrienyl.

Durch Cholesteryloxy substituiertes aliphatisches Hydrocarbyl R₈ ist vorzugsweise in ω-Stellung durch Cholesteryloxy substituiertes aliphatisches Hydrocarbyl z.B. ω-Cholesteryloxy-n-hexadecyl.

Ein bivalenter aliphatischer Kohlenwasserstoffrest X mit bis zu 23 C-Atomen ist ein Rest, bei dem die beiden freien Valenzen von ein- und demselben oder von verschiedenen, vorzugsweise endständigen C-Atomen ausgehen können, z.B. ein solcher Alkinylen-oder vorzugsweise Alkylen- oder Alkenylenrest. Vorzugsweise hat ein solcher Hydrocarbylrest 11-17 C-Atome. Ein solcher Alkylenrest ist z.B. geradkettig, wie n-Undecylen, n-Tridecylen, n-Heptadecylen oder insbesondere n-Pentadecylen. Ein solcher Alkenylenrest hat vorzugsweise 17 C-Atome und ist z.B. cis- oder trans-9-Heptadecenyl, -9,12-heptadecadienyl oder -9,11,13-heptadecatrienyl.

Triniederalkyl-silyl R₃ oder R₄ ist z.B. Trimethyl-silyl.

Durch Niederalkoxy substituiertes Alkoxy mit bis zu 20 C-Atomen R₃ oder R₄ ist insbesondere Niederalkoxy-niederalkoxy, wie hauptsächlich 2-Niederalkoxy-ethoxy, z.B. 2-Methoxy-ethoxy.

Die vor- und nachstehend mit dem Präfix "Nieder-" bezeichneten Reste enthalten bis und mit 7, vorzugsweise bis und mit 4 C-Atome.

Die Verbindungen der Formel I besitzen wertvolle pharmakologische Eigenschaften. Sie können z.B. in der photodynamischen Chemotherapie eingesetzt werden. Dies lässt sich z.B. durch folgende Versuchsanordnung demonstrieren:

Die Verbindungen der Formel I werden intravenös oder topisch an Mäuse oder Ratten mit transplantablen oder chemisch induzierten Tumoren oder an Nacktmäuse mit Transplantaten von menschlichen Tumoren verabreicht. Z.B. werden BALB/c Mäuse 7-10 Tage nach der Transplantation eines Meth-A Sarcoms mit einer Verbindung der Formel I behandelt. In der Zeitspanne von 1 bis 7 Tagen nach der einmaligen Verabreichung einer Verbindung der Formel I wird der Tumor selektiv mit einem "Argon-pumped-dye-Laser" oder Titan-Saphir Laser bei einer Wellenlänge von z.B. 678 nm mit einer Lichtdosis von 50-300, z.B. 100-200, Joule/cm² bestrahlt. (Die jeweils erforderliche Wellenlänge hängt von der spezifisch ausgewählten Verbindung der Formel I ab.) In dieser Versuchsanordnung bewirken die Verbindungen der Formel I in einer Dosierung von etwa 0,01 bis 3 mg/kg, z.B. bei 0,1 bis 1,0 mg/kg, ein vollständiges Verschwinden des Tumors (Nekrotisierung des Tumors nach 3-7 Tagen und vollständige Heilung der Mäuse nach etwa 21 Tagen). Unter diesen Bedingungen bewirken die Verbindungen der Formel I keine unerwünschte Lichtsensibilisierung der gesunden Teile der Tiere. Während einer Nachbeobachtungszeit von 3 Monaten wird unter diesen Bedingungen weder eine allgemeine Photosensibilisierung noch ein erneutes Tumorwachstum beobachtet.

Im Rahmen der photodynamischen Therapie können die Verbindungen der Formel I auch zur Behandlung virusbedingter Erkrankungen, wie bei AIDS bedingtem Kaposi Sarkom, zur Virusinaktivierung (z.B. im Falle von Herpes, AIDS) von Blutkonserven, zur Behandlung von Hauterkrankungen, wie Psoriasis oder Akne Vulgaris, und bei Arteriosklerose eingesetzt werden. Ausserdem sind sie in der Diagnostik, z.B. zum Auffinden von Tumoren, verwendbar.

Bevorzugt sind Verbindungen der Formel I, worin R₇ Cholesteryloxy, unsubstituiertes oder durch Cholesteryloxy substituiertes aliphatisches Hydrocarbyloxy mit bis zu 24 C-A-tomen im aliphatischen Teil oder, wenn m für 1 und n für 0 steht, aliphatisches Hydrocarbyl mit 6-24 C-Atomen bedeutet, oder R₁ für einen Rest der obigen Formel III steht, worin X einen bivalenten aliphatischen Kohlenwasserstoffrest mit bis zu 23 C-Atomen bedeutet, Y für Sauerstoff oder die Gruppe -NH- steht, und R₈ für Cholesteryl, aliphatisches Hydrocarbyl mit bis zu 24 C-Atomen oder unsubstituiertes oder durch Cholesteryloxy substituiertes aliphatisches Hydrocarbyl mit bis zu 24 C-Atomen im aliphatischen Teil steht, und die übrigen Substituenten die obengenannten Bedeutungen haben, mit Ausnahme der Verbindung der Formel I, worin M für Zinn, R₁ und R₂ je für den Rest -OSi(C₆H₁₃)₃ und R₃ und R₄ je für teil. Butyl stehen.

Besonders bevorzugt sind Verbindungen der Formel I, worin M für Germanium steht, R₁ und R₂ je einen und denselben Rest der Formel II, worin m für 0 oder 1 steht, n für eine ganze Zahl von und mit 0 bis und mit 20 steht, R₅ und R₆ unabhängig voneinander je für Niederalkyl oder Phenyl stehen und R₇ für Cholesteryloxy, Alkyl mit 6-24 C-Atomen, Alkoxy mit bis zu 24 C-Atomen, Alkenyloxy mit bis zu 24 C-Atomen oder für ω-Cholesteryloxy-alkoxy mit bis zu 24 C-Atomen im Alkoxyteil steht, oder einen und denselben Rest der Formel III, worin m für 0 oder 1 steht, X für Alkylen mit bis zu 23 C-Atomen steht, Y für Sauerstoff steht, R₅ und R₆ je für Phenyl oder Niederalkyl stehen und R₈ für Cholesteryl steht, bedeuten, R₃ für Wasserstoff, Triniederalkylsilyl oder für unsubstituiertes oder durch Niederalkoxy substituiertes Alkoxy mit bis zu 20 C-Atomen steht, und R₄ Wasserstoff bedeutet.

Hauptsächlich bevorzugt sind Verbindungen der Formel I, worin M für Germanium steht, R₁ und R₂ je einen und denselben Rest der Formel II, worin m für 0 oder 1 steht, n für eine ganze Zahl von und mit 0 bis und mit 5 steht, R₅ und R₆ je für Phenyl oder Niederalkyl stehen und R₇ für Cholesteryloxy, Alkoxy mit bis zu 18 C-Atomen, C₁₈-Alkenyloxy, Alkyl mit 6 bis 18 C-Atomen, ω-Cholesteryloxy-C₁₂₋₁₈-alkoxy, Phenyl, Cholestan-3-yloxy, 4-(2,3,4,6-Tetra-O-acetyl-β-D-glucopyranos-1-yloxy)-phenoxy, C₁₀₋₁₄-Alkylthio oder Niederalkanoyloxy steht, oder worin R₁ und R₂ je einen und denselben Rest der Formel III bedeuten, worin m für 1, X für C₁₁₋₁₇-Alkylen, Y für Sauerstoff, R₅ und R₆ je für Phenyl und R₈ für Cholesteryl stehen, R₃ Wasserstoff oder durch Niederalkoxy substituiertes Niederalkoxy und R₄ Wasserstoff bedeuten.

In erster Linie bevorzugt sind Verbindungen der Formel I, worin M für Germanium steht, R₁ und R₂ je einen und denselben Rest der Formel II, worin m für 0 oder 1 steht, n für 0, 2, 3 oder 5 steht, R₅ und R₆ je für Phenyl, Methyl oder n-Hexyl stehen und R₇ für Cholesteryloxy, Methoxy, n-Hexadecyloxy, cis-9-Octadecenyloxy, n-Hexyl, 1,1,2-Trimethylpropyl, n-Octadecyl, ω-Cholesteryloxy-n-hexadecyloxy, Phenyl, 5-α-Cholestan-3-β-yloxy, 4-(2,3,4,6-Tetra-O-acetyl-β-D-glucopyranos-1-yloxy)-phenoxy, n-Dodecylthio oder Acetoxy steht, oder worin R₁ und R₂ je einen und denselben Rest der Formel III bedeuten, worin m für 1, X für n-Pentadecylen, Y für Sauerstoff, R₅ und R₆ je für Phenyl und R₈ für Cholesteryl stehen, R₃ Wasserstoff oder 2-Methoxy-ethoxy und R₄ Wasserstoff bedeuten.

Ganz besonders bevorzugt sind die in den Beispielen beschriebenen Verbindungen der Formel I.

Die Verbindungen der Formel I werden nach an sich bekannten Verfahren hergestellt. Das erfindungsgemässe Verfahren ist dadurch gekennzeichnet, dass man eine Verbindung der Formel V, 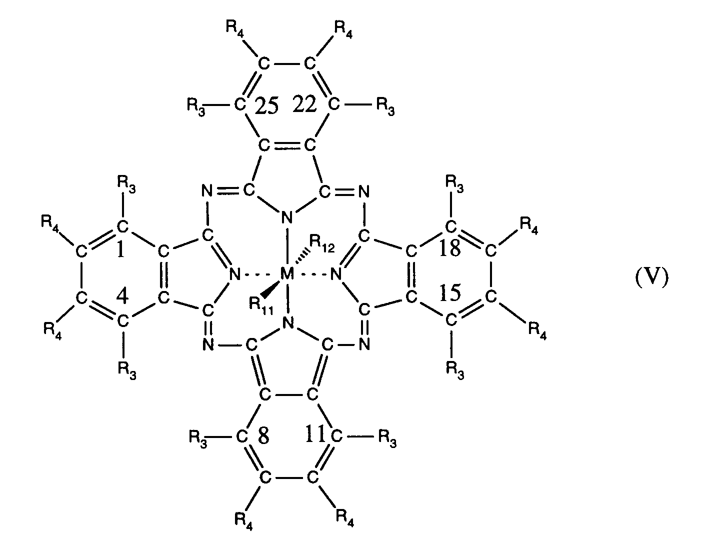 worin R₁₁ für Hydroxy oder Halogen steht und R₁₂ die Bedeutung von R₁₁ hat, wenn M ein Element der vierten Hauptgruppe ist, oder R₁₂ fehlt, wenn M ein Element der dritten Hauptgruppe oder Ruthenium ist, und die übrigen Symbole die obengenannten Bedeutungen haben, mit einer Verbindung der Formel VI,

R₁-H (VI)

worin R₁ die obengenannten Bedeutungen hat, oder mit einem reaktionsfähigen Derivat davon mit aktivierter OH-Gruppe umsetzt.

Halogen R₁₁ ist vorzugsweise Chlor.

Ein reaktionsfähiges Derivat einer Verbindung der Formel VI mit aktivierter OH-Gruppe ist ein Derivat, in welchem die terminale OH-Gruppe in reaktionsfähiger veresterter Form vorliegt, z.B. in Form von Estern mit Mineralsäuren, wie Halogenwasserstoffsäuren oder Schwefelsäure, oder mit Sulfonsäuren, wie p-Toluolsulfonsäure. Ein bevorzugtes reaktionsfähiges Derivat ist ein Halogenid, wie insbesondere Chlorid.

Vorzugsweise setzt man eine Verbindung der Formel V, worin R₁₁ für Hydroxy steht, mit einer Verbindung der Formel VI mit terminaler OH-Gruppe, das heisst mit einer Verbindung der Formel R₁-H um. Alternativ kann man auch eine Verbindung der Formel V, worin R₁₁ für Hydroxy steht, mit einem reaktionsfähigen Derivat einer Verbindung der Formel VI mit aktivierter OH-Gruppe, z.B. einem Halogenid, umsetzen.

Die Reaktion wird in den meisten Fällen vorzugsweise in einem geeigneten inerten Lösungsmittel, z.B. einem aromatischen Kohlenwasserstoff, wie Toluol oder Benzol, einem Ether, wie insbesondere cyclischen Ether, wie vorzugsweise Dioxan, oder einem Lösungsmittelgemisch durchgeführt, wobei man auch von einer Suspension der Verbindung der Formel V in dem genannten Lösungsmittel ausgehen kann. Die Reaktionstemperatur hängt unter anderem davon ab, ob R₁₁ für Wasserstoff oder Halogen steht und ob die Verbindung der Formel VI in aktivierter Form vorliegt. Normalerweise liegt die Reaktionstemperatur zwischen Raumtemperatur und etwa dem Siedepunkt des verwendeten Lösungsmittels oder Lösungsmittelgemisches, wobei, wenn erwünscht oder erforderlich, auch unter Druck und/oder einer Inertgasatmosphäre, z.B. unter Argon, gearbeitet werden kann. Zur Herstellung von Verbindungen der Formel I, worin R₁ einen Rest der Formel II bedeutet, worin m und n je für 0 und R₇ für aliphatisches Hydrocarbyloxy mit bis zu 6, insbesondere 1-3, C-Atomen stehen, kann man den entsprechenden Alkohol der Formel R₇-H nicht nur als Reagenz, sondern gleichzeitig auch als Lösungsmittel verwenden.

Die Ausgangsmaterialien der Formel V und VI sind teilweise bekannt oder können wie oder analog wie in den Beispielen beschrieben hergestellt werden.

Die Erfindung betrifft auch die Verwendung der Verbindungen der Formel I, vorzugsweise in Form pharmazeutischer Zusammensetzungen, zur therapeutischen Behandlung des menschlichen oder tierischen Körpers, insbesondere zur Behandlung von Tumoren durch photodynamische Chemotherapie. Die Erfindung betrifft insbesondere eine Methode zur Behandlung von Tumoren in Warmblütern einschliesslich des Menschen durch photodynamische Chemotherapie, wobei eine in der genannten Chemotherapie wirksame Dosis einer Verbindung der Formel I an einen solchen Warmblüter verabreicht wird, der eine solche Behandlung braucht. Die Dosierung des Wirkstoffs hängt unter anderem von der Art der Krankheit, z.B. der Grösse des Tumors, der Art und Grösse der zu behandelnden Spezies und der Applikationsweise ab. Beispielsweise verabreicht man, vorzugsweise intravenös, an einen Warmblüter von etwa 70 kg Körpergewicht eine tägliche Dosis von 1 mg bis 100 mg einer Verbindung der Formel I.

Die Erfindung betrifft im weiteren pharmazeutische Zusammensetzungen, welche die Verbindungen der vorliegenden Erfindung als Wirkstoffe enthalten, sowie Verfahren zur Herstellung dieser Zusammesetzungen. Die Erfindung betrifft insbesondere pharmazeutische Zusammensetzungen zur Anwendung bei der photodynamischen Chemotherapie von Tumoren, welche eine in der genannten Chemotherapie wirksame Dosis einer Verbindung der Formel I zusammen mit pharmazeutischem Trägermaterial enthalten.

Bei den erfindungsgemässen pharmazeutischen Zusammensetzungen handelt es sich z.B. um solche zur parenteralen, wie insbesondere intravenösen, Verabreichung an Warmblüter.

Für die Anwendung zur photodynamischen Chemotherapie sind solche Zusammensetzungen besonders bevorzugt, die sich im Tumorgewebe anreichem. Zu diesem Zweck besonders geeignet sind Liposomenformulierungen, z.B. analog den in der Europäischen Patentanmeldung mit der Veröffentlichungsnummer 451103 beschriebenen. Diese enthalten neben einem Gewichtsteil Wirkstoff insbesondere 25-100 Gewichtsteile 1-n-Hexadecanoyl-2-(9-cis-octadecenoyl)-3-sn-phosphatidyl-cholin (POPC), 0 bis 75 Gewichtsteile 1,2-Di-(9-cis-octadecenoyl)-3-sn-phosphatidyl-S-serin (OOPS) und 0 bis 500 Gewichtsteile Lactose.

Die nachfolgenden Beispiele illustrieren die oben beschriebene Erfindung, schränken jedoch deren Umfang in keiner Weise ein. Temperaturen sind in Celsiusgraden angegeben. Die in den nachstehenden Beispielen verwendete Bezifferung der C-Atome des Phthalocyanin-Ringsystems entspricht der in Formel I angegebenen.

Beispiel 1: 1 g (1,62 mMol) Dihydroxygermanium-phthalocyanin (hergestellt nach Joyner et.al., Inorg. Chem. 1, 236 [1962]) werden in 100 ml Dioxan suspendiert und mit 1,89 g (3,24 mMol) Diphenylcholesteryloxysilanol versetzt. Man kocht das Gemisch während 48 Stunden unter Rückfluss. Nach 20 Stunden werden ca. 20 ml Dioxan abdestilliert. Man lässt das Reaktionsgemisch erkalten und dampft zur Trockne ein. Der Rückstand wird mit 600 ml Hexan versetzt, 15 Minuten gerührt und abgenutscht. Der Filterrückstand wird gut mit Hexan gewaschen und an Aluminiumoxid (Alox B) chromatographiert, wobei man als Laufmittel nacheinander Hexan-Tetrahydrofuran (9:1 und 1:1) und reines Tetrahydrofuran verwendet. Die Produkte enthaltenden Fraktionen werden vereinigt und aus Methylenchlorid-Hexan umkristallisiert. Man erhält Bis-(Diphenylcholesteryloxysiloxy)-germaniumphthalocyanin in Form blauer Kristalle; Smp. 247-276°C, NMR (CDCl₃): δ = 9.50 (m,8H); 8.32 (m,8H); 6.70 (m,4H); 6.28 (m,8H); 4.90 (m,8H); 4.64 (m,2H); 2.1-0 (mehrere m's, Cholesterin-H) ppm, UV: 685 nm (ε = 269000 in CH₂Cl₂).

Das als Ausgangsmaterial eingesetzte Dihydroxygermanium-phthalocyanin wird folgendermassen hergestellt:

Stufe 1.1: 100 g (0,78 Mol) Phthalodinitril in 200 ml Chinolin (Fluka puriss.) werden in einem Sulfierkolben bei Raumtemperatur mit 40 g (0,168 Mol) Germaniumtetrachlorid versetzt. Man erwärmt das Reaktionsgemisch langsam auf 220°C Badtemperatur und rührt während 9 Stunden bei dieser Temperatur. Danach lässt man das Gemisch abkühlen und filtriert über eine Nutsche das Produkt ab. Der Rückstand wird mit Dimethylformamid und Aceton gewaschen. Die blauen Kristalle werden in ein Soxhlet transferiert, über Nacht zuerst mit Dimethylformamid, dann 6,5 Stunden mit Xylol und nochmals über Nacht mit Aceton extrahiert. Nach Trocknen am Hochvakuum bei 70° C erhält man Dichlorgermanium-phthalocyanin [R.D. Joyner and M.E. Kenney, J.Am.Chem.Soc. 82, 5790 (1960)]. Die Substanz ist in allen Lösungsmittel praktisch unlöslich.

Stufe 1.2: 27,8 g Dichlorgermanium-phthalocyanin werden in einem 2 Liter Autoklaven mit 500 ml Pyridin und 500 ml 30proz. Ammoniaklösung (techn.) versetzt und 12 Stunden auf 100° erwärmt. Dabei wird ein Druck von 6 bar erreicht. Nach Abkühlen wird der Rückstand über eine Nutsche abfiltriert, fünfmal mit Wasser aufgeschlämmt und neutral gewaschen. Nach weiterem Waschen mit Ethanol (2mal), Aceton und Diethylether (je 1mal) trocknet man das erhaltene Pulver am Hochvakuum bei 70° über Nacht. Man erhält Dihydroxygermanium-phthalocyanin [R.D. Joyner and M.E. Kenney, J. Am. Chem. Soc. 82, 5790 (1960)] als blaues, mikrokristallines Pulver. Die Substanz ist in allen Lösungsmittel praktisch unlöslich.

Das Reagenz, Diphenylcholesteryloxysilanol, wird folgendermassen hergestellt:

### Stufe 1.3:

Variante a: (Mitsunobu Bedingungen; Reviewartikel in Synthesis 1981, 1; J. Org. Chem. 56, 670 [1991]):
Eine Lösung bestehend aus 5 g (23,1 mMol) Diphenylsilanol, 4,47 g (11,55 mMol) Cholesterin, 6,12 g (23,1 mMol) Triphenylphosphin und 4,7 ml (23, 1 mMol) Azodicarbonsäurediisopropylester in 100 ml abs. Tetrahydrofuran wird während 3,5 Tagen bei 50°C gerührt. Das Reaktionsgemisch wird weitgehend eingeengt und der Rückstand mehrmals an Kieselgel chromatographiert, wobei man als Laufmittel Hexan-Essigsäureethylester (2,5:1) verwendet. Man erhält Diphenylcholesteryloxysilanol als farblose Kristalle; Smp. 146-150°C, NMR (CDCl₃): δ = 7.68 (m,4H); 7.40 (m,8H); 5.25 (m,1H); 3.78 (m,1H); 2.6-0.5 (mehrere m's, Cholesterin-H) ppm.

Variante b: (J. Chem. Soc. Chem. Commun. 1987, 325): Zu einer Lösung von 4,19 ml (20 mMol) Diphenyldichlorsilan in 100 ml abs. Benzol tropft man innerhalb von 45 Minuten unter Feuchtigkeitsausschluss (Argonballon) bei Raumtemperatur eine Lösung aus 7,97 g Cholesterin und 1,62 ml Pyridin in 150 ml absolutem Benzol. Nach kurzer Zeit bildet sich ein Niederschlag von Pyridinhydrochlorid. Nach beendigtem Zutropfen wird das Gemisch auf ca. 60° erwärmt und weitere 4-5 Stunden nachgerührt. Nach beendigter Reaktion (Dünnschichtkontrolle) wird der Kolbeninhalt mit Essigsäureethylester in einen Scheidetrichter transferiert und die organische Phase mit Wasser und gesättigter Kochsalzlösung gewaschen. Nach Entfernung des Lösungsmittels chromatographiert man den Rückstand an Kieselgel (Hexan-Tetrahydrofuran [2,5/1]). Man erhält Diphenylcholesteryloxysilanol, welches mit dem in Stufe 1.1 beschriebenen identisch ist.

Beispiel 2: 200 mg (0,181 mMol) Bis-(Diphenylhydroxysiloxy)-germanium-phthalocyanin (hergestellt nach Joyner et.al., J. Inorg. Nucl. Chem. 24, 299 [1962]) werden unter Argon zusammen mit 500 mg Cholesterin in einem Bombenrohr während 65 Stunden auf 165°C erhitzt. Nach Erkalten wird der Rückstand an Alox B chromatographiert (Laufmittel: CH₂Cl₂-Hexan [1:1]) und aus CH₂Cl₂-Hexan umkristallisiert. Man erhält Bis-(Diphenylcholesteryloxysiloxy)-germanium-phthalocyanin in Form blauer Kristalle mit identischen Eigenschaften wie das nach Beispiel 1 erhaltene Material.

Das Ausgangsmaterial erhält man folgendermassen:

Stufe 2.1: 0,5 g (0,809 mMol) Germaniumdihydroxy-phthalocyanin und 0,524 g (2,42 mMol) Diphenylsilandiol in 10 ml Benzol werden 4 Stunden unter Rückfluss gekocht. Nach Erkalten der Reaktionslösung wird über einer Fritte abfiltriert und der Rückstand (d.h. nicht umgesetztes Germaniumdihydroxy-phthalocyanin) mit Diethylether gewaschen. Nach Entfernung des Lösungsmittels wird der Rückstand in CH₂Cl₂/CHCl₃/Dioxan gelöst und bei Normaldruck das Lösungsmittelgemisch bis auf ca. 20 ml eingeengt. Beim Stehenlassen bei Raumtemperatur kristallisiert Bis-(Diphenylhydroxysiloxy)-germaniumphthalocyanin als Dioxan Komplex aus (Joyner et. al., J. Inorg. Nucl. Chem. 24, 299 [1962]); NMR (CDCl₃): δ = unter anderem 9.50 (m, 8H); 8.36 (m,8H); 6.69 (m,4H); 6.33 (m,8H); 5.04 (m,8H) ppm.

### Beispiel 3:

Variante a: 1,16 g (1,875 mMol) Germaniumdihydroxy-phthalocyanin und 1,65 g Rohprodukt aus Stufe 3.1, enthaltend Diphenylhexadecyloxysilanol, werden in 100 ml Dioxan während 18 Stunden unter Rückfluss gekocht. Man lässt die Lösung erkalten und filtriert über eine Fritte ab. Der Rückstand auf der Fritte beträgt nach Trocknen am Hochvakuum 662 mg und ist nicht umgesetztes Ausgangsmaterial. Das Filtrat wird zu einem öligen Rückstand eingeengt und am Hochvakuum getrocknet. Dabei kristallisiert ein Teil der Substanz aus. Bei Zugabe von Pentan entsteht ein sehr feiner, mikrokristalliner Niederschlag des gewünschten Produkts. Der Niederschlag wird abfiltriert, mit Methanol gewaschen und getrocknet. Umkristallisation einer Probe aus heissem t-Butanol ergibt Kristalle von Bis-(Diphenylhexadecyloxy-siloxy)-germanium-phthalocyanin; Smp. 87-89°, NMR (CDCl₃): δ = 9.62 (m,8H); 8.35 (m,8H); 6.68 (m,4H); 6.31 (m,8H); 4.96 (m,8H); 1.5-0 (restliche H's, von Hexadecylseitenkette) ppm.

Das Ausgangsmaterial für Variante a erhält man folgendermassen:

Stufe 3.1: 1 ml (1,22 g; 4,8 mMol) Diphenylchlorsilan und 4,8 g Polyhünigbase (Diisopropylaminomethyl-polystyrol) werden in 20 ml Toluol vorgelegt. Bei Raumtemperatur 1,167 g (4,8 mMol) Hexadecanol gelöst in 25 ml Toluol langsam während 50 Minuten zugetropft. Das Reaktionsgemisch wird während 4 1/4 Stunden bei Raumtemperatur gerührt. Danach nutscht man von der polymeren Base ab, transferiert das Filtrat in einen Scheidetrichter, verdünnt mit Essigsäureethylester und wäscht die örganische Phase je zweimal mit gesättigter Natriumhydrogencarbonat-Lösung und Sole. Nach Trocknen über Na₂SO₄ und Einengen des Lösungsmittels verbleibt ein öliger, zum Teil fester Rückstand, der mit Hexan digeriert wird. Die Lösung wird abgenutscht, eingeengt und über Nacht am Hochvakuum getrocknet. Das Rohprodukt enthält u.a. die gewünschte Verbindung Diphenylhexadecyloxysilanol und wird ohne Reinigung in Beispiel 3, Variante a, eingesetzt.

Variante b: 2 g (3,24 mMol) Dihydroxygermanium-phthalocyanin werden mit 2 Aequivalenten Diphenylhexadecyloxysilanol in 210 ml Dioxan während 39 Stunden am Rückfluss gekocht. Nach 24 Stunden werden 500 mg Dihydroxygermanium-phthalocyanin nachgegeben. Man lässt das Reaktionsgemisch erkalten und chromatographiert den Rückstand an basischem Aluminiumoxid (Alox B), wobei man als Laufmittel nacheinander reines Hexan und Hexan-Essigsäureethylester (4:1) verwendet. Die Produkt enthaltenden Fraktionen werden vereinigt und eingeengt. Der Rückstand wird aus Pentan bei -20°C umkristallisiert. Man erhält blaue Kristalle von Bis-(Diphenylhexadecyloxy-siloxy)-germanium-phthalocyanin.

Das Reagenz Diphenylhexadecyloxysilanol für Variante b wird unter Mitsunobu Bedingungen in Analogie zu Stufe 1.3, Variante a, hergestellt; NMR (CDCl₃): 7.8-7.0 (2 m's, 10H); 3.83 (t,2H); 1.8-0.7 (diverse m's, Hexadecyl-H) ppm.

Beispiel 4: Analog Beispiel 1 erhält man aus 2,99g (6,4 mMol) Diphenyl-cis-9-octadecenyloxysilanol und 2 g (3,2 mMol) Dihydroxygermanium-phthalocyanin Bis-(Diphenyl-cis-9-octadecenyloxy-siloxy)-germanium-phthalocyanin; UVₘₐₓ: 674 nm in Methanol.

Das Ausgangsmaterial erhält man folgendermassen:

Stufe 4.1: Diphenyl-cis-9-octadecenyloxysilanol wird unter Mitsunobu Bedingungen aus 2 g (9,25 mMol) Diphenylsilandiol, 2,08 ml (4,62 mMol) cis-9-Octadecen-1-ol, 2,45 g (9,25 mMol) Triphenylphosphin und 1,88 ml (9,25 mMol) Azodicarbonsäurediisopropylester in 40 ml Tetrahydrofuran in Analogie zu Stufe 1.3., Variante a, hergestellt.

Beispiel 5: Analog Beispiel 1 wird aus 110 mg (0,235 mMol) Diphenyl-octadecyloxysilanol und 73 mg (0,117 mMol) Dihydroxy-germanium-phthalocyanin Bis-(Diphenyl-octadecyloxy-siloxy)-germanium-phthalocyanin hergestellt. Diphenyl-octadecyloxysilanol wird unter Mitsunobu Bedingungen aus 2 g (9,25 mMol) Diphenylsilandiol, 2,5 g (9,25 mMol) 1-Octadecanol, 2,99 g (11,1 mMol) Triphenylphosphin und 1,93 ml (11,1 mMol) Azodicarbonsäurediethylester in 40 ml Benzol analog zu Stufe 1.3., Variante a, hergestellt.

Beispiel 6: [In Analogie zu einer Vorschrift von Wheeler et.al., J.Am.Chem.Soc. 106, 7404 (1984)] 3,3 g (9,5 mMol) Dimethyl-octadecyl-chlor-silan und 0,5 g (0,8 mMol) Dihydroxygermanium-phthalocyanin in 50 ml Pyridin werden 2 Stunden bei 50° gerührt. Die Lösung wird abgekühlt und über eine Nutsche abfiltriert. Das Lösungsmittel wird unter Vakuum entfernt und der Rückstand mit 250 ml Hexan versetzt. Der ausgefallene Niederschlag wird abgenutscht und zuerst mit Hexan, dann portionenweise mit 200 ml Aceton-Wasser (1:1) gewaschen. Der Rückstand wird über Nacht am Hochvakuum getrocknet, danach im Soxhlet mit Toluol extrahiert, vom Lösungsmittel befreit und getrocknet, worauf man Bis-(Dimethyl-octadecyl-siloxy)-germanium-phthalocyanin erhält; NMR (Pyridin-d5): δ = 9.8-9.9 (m,8H); 8.3-8.5 (m,8H): aromatische H's; 0.75-1.46 (diverse m's); 0.45(m); -0.03(m); -0.88 (m); -1.91(m); -2.48 (m, 4xCH₃: Silylseitenketten) ppm.

Alternativ kann man Bis-(Dimethyl-octadecyl-siloxy)-germanium-phthalocyanin durch Kochen in Dioxan von Dihydroxygermanium-phthalocyanin und Dimethyl-octadecyl-silanol (beschrieben in Helv. Chim. Acta 59, 717 [1976]) herstellen.

Beispiel 7: In Analogie zu Beispiel 1 erhält man aus 0,755 g (1,22 mMol) Dihydroxygermanium-phthalocyanin und 1,646 g (2,44 mMol) 5-Cholesteryloxy-3-oxa-pentyloxy)diphenyl-silanol in 80 ml Dioxan Bis-[(5-cholesteryloxy-3-oxa-pentyloxy)-diphenylsiloxy] -germanium-phthalocyanin. Das Silanol wird unter Mitsunobu Bedingungen analog Stufe 1.3., Variante a, aus 1 g (2,1 mMol) 5-Cholesteryloxy-3-oxa-pentan-1-ol, 0,45 g (2,1 mMol) Diphenylsilandiol, 0,661 g (2,52 mMol) Triphenylphosphin und 0,439 ml (2,52 mMol) Azodicarbonsäurediethylester hergestellt.

Alternativ kann man 5-Cholesteryloxy-3-oxa-pentyloxy)-diphenyl-silanol auch nach Stufe 1.3, Variante b, aus 0,441 ml (2,1 mMol) Diphenylchlorsilan, 0,184 ml (1,1 Aequiv.) Pyridin und 1 g (2,1 mMol) 5-Cholesteryloxy-3-oxa-pentan-1-ol in 50 ml THF herstellen.

Die Ausgangsverbindung für das Silanol, 5-Cholesteryloxy-3-oxa-pentan-1-ol, erhält man folgendermassen (vgl. Bull. Soc. Chim. France 1960, 297; J. Chem. Soc. 1962, 178):

Sufe 7.1: 50 g (0,129 mMol) Cholesterin werden in 250 ml Pyridin gelöst und mit Eis/Wasser auf ca. 5°C abgekühlt Unter Argon gibt man portionenweise 49 g (0,258 Mol) Tosylchlorid dazu. Nach beendigter Zugabe rührt man weitere 15 Minuten unter Kühlung. Danach wird das Eisbad entfernt und die Reaktion 2 Tage bei Raumtemperatur ausgerührt. Die erhaltene feine Suspension wird auf 1,2 Liter Eiswasser gegossen und während 30 Minuten verrührt. Der Niederschlag wird abgenutscht, gut mit H₂O gewaschen und am Hochvakuum bei 40° getrocknet. Die Substanz, die noch viel Pyridinhydrochlorid enthält, wird in CH₂Cl₂ gelöst und die organische Phase 2mal mit Wasser, 1mal mit 1N Salzsäure und nochmals mit Wasser gewaschen. Nach Trocknen über Na₂SO₄ und Einengen am Rotationsverdampfer kristallisiert man den Rückstand aus CH₂Cl₂/Hexan um. Man erhält Cholesteryltosylat; Smp. 130-131°.

Stufe 7.2: 20 g (36,98 mMol) Cholesteryltosylat und 133 ml (44,38 mMol) Diethylenglykol in 275 ml Dioxan (Fluka puriss.) werden während 2 3/4 Stdn. unter Rückfluss gekocht. Die erkaltete Lösung wird weitgehend eingeengt, der Rückstand in Wasser aufgenommen und 3mal mit Diethylether extrahiert. Die organische Phase wird 2mal mit Wasser und 1mal mit Sole gewaschen. Nach Trocknen über Na₂SO₄ und Entfemen des Lösungsmittels wird der Rückstand an Kieselgel in Hexan-Essigsäureethylester (2,5 : 1) chromatographiert. Man erhält 5-Cholesteryloxy-3-oxa-pentan-1-ol als wachsartiges Produkt.

Beispiel 8: Analog Beispiel 1 erhält man aus 40 mg (0,064 mMol) Dihydroxygermaniumphthalocyanin und 50 mg (0,132 mMol) Diphenyl-(3,6,9,12,15-pentaoxa-hexadec-1-yloxy)-silanol in 8 ml Dioxan Bis-[Diphenyl-(3,6,9,12,15-pentaoxa-hexadec-1-yloxy)siloxy]-germanium-phthalocyanin; UVₘₐₓ = 676 nm (CH₂Cl₂).

Das Ausgangsmaterial erhält man folgendermassen:

Stufe 8.1: 19,02 g (0,116 Mol) Triethylenglykolmonomethylether und 21 ml (20,54 g = 0,26 Mol) Pyridin in 110 ml Benzol werden zum Rückfluss erhitzt und tropfenweise mit 19 ml (31 g = 0,26 Mol) Thionylchlorid versetzt. Man kocht weitere 20 Stunden unter Rückfluss. Nach Abkühlen der Reaktionslösung versetzt man diese unter Rühren mit einem Gemisch bestehend aus 5 ml konzentrierter Salzsäure und 20 ml Wasser. Die organische Phase wird abgetrennt und mehrmals mit Wasser und Sole gewaschen. Das Lösungsmittel wird über eine Vigreuxkolonne weitgehend abdestilliert, der Rückstand an Kieselgel in Hexan-Essigsäureethylester (4:1) gereinigt. Die Produkte enthaltenden Fraktionen werden eingeengt und der Rückstand destilliert. Man erhält 3,6,9-Trioxadecylchlorid (J. Am. Chem. Soc. 89, 7017 (1967)); Sdp. 76°C/2mbar, NMR (CDCl₃): 3.8-3.5 (m, 12H); 3.37 (s,3H).

Stufe 8.2: 10 ml Diethylenglykol werden unter Argon mit 630 mg (27,4 mMol) Natrium versetzt und 1 Stunde auf 100°C erhitzt. Bei dieser Temperatur werden 5 g (27,4 mMol) 3,6,9-Trioxadecylchlorid tropfenweise mittels einer Spritze zugegeben. Die Reaktion wird über Nacht bei 100°C ausgerührt. Nach Erkalten wird die Reaktionslösung in CH₂Cl₂ aufgenommen, mit Wasser und Sole gewaschen und über Na₂SO₄ getrocknet. Nach Entfernung des Lösungsmittels erhält man 3,6,9,12,15-Pentaoxa-hexadecanol als Öl (Liebigs Ann. Chem. 1980, 858); NMR(CDCl₃): 3.8-3.5 (m, 20H); 3.75 (s,3H).

Stufe 8.3: Unter Mitsunobu Bedingungen erhält man analog Stufe 1.3., Variante a, aus 2 g (7,83 mMol) 3,6,9,12,15-Pentaoxa-hexadecanol, 3,39 g (21,63 mMol) Diphenylsilandiol, 4,11 g (15,66 mMol) Triphenylphosphin und 3,2 ml (3,17 g; 15,7 mMol) Azodicarbonsäurediisopropylester in 70 ml Tetrahydrofuran Diphenyl-(3,6,9,12,15-pentaoxa-hexadec-1-yloxy)-silanol; NMR (CDCl₃): 7.1-7.8(m's, Phenyl-H); 3.4-3.7(m, OCH₂-CH₂); 3.35 (s, 3H, OCH₃).

Alternativ kann man die gleiche Verbindung auch nach Stufe 3.1. aus Diphenylchlorsilan und 3,6,9,12,15-Pentaoxa-hexadecanol in Gegenwart von Pyridin erhalten.

Beispiel 9: Analog Beispiel 1 erhält man aus Dihydroxygermanium-phthalocyanin und Diphenyl-(ω-cholesteryloxycarbonyl-n-pentadec-1-yloxy)-silanol Bis-[Diphenyl-(ω-cholesteryloxycarbonyl-n-pentadec-1-yloxy)-siloxy] -germanium-phthalocyanin. Das verwendete Silanol wird unter Mitsunobu Bedingungen analog Stufe 1a hergestellt. Die Herstellung der Ausgangsverbindung für das Silanol, Cholesteryl-1-hydroxy-palmitat, erfolgt unter Verwendung der Tetrahydropyranylether-Schutzgruppe mit anschliessender Abspaltung nach Analogievorschriften aus der Literatur (Lipids 14, 816 [1979]; Synthetic Commun. 16, 1423 [1986]).

Beispiel 10: Analog den in dieser Anmeldung beschriebenen Verfahren erhält man die folgenden Verbindungen:
Di-(ω-Cholesteryloxy-n-hexadecyloxy)-germanium-phthalocyanin,
Bis-[(ω-Cholesteryloxy-n-hexadecyloxy)-diphenyl-siloxy]-germanium-phthalocyanin
Bis-[(8-Cholesteryloxy-3,6-dioxa-octyloxy)-diphenyl-siloxy]-germanium-phtalocyanin und
Di-(8-Cholesteryloxy-3,6-dioxa-octyloxy)-germanium-phthalocyanin.

Beispiel 11: Eine Mischung aus 242 mg (0,2 mMol) 1,4,8,11,15,18,22,25-Octakis-(2-methoxy-ethoxy)-dihydroxy-germanium-phthalocyanin, 350 mg (0,6 mMol) Diphenylcholesteryloxysilanol (siehe Stufe 1.3), 3 ml Pyridin und 30 ml abs. Toluol wird eine Stunde am Rückfluss erhitzt. Anschliessend wird zur Trockne verdampft, der Rückstand in Diethylether gelöst und 4mal mit Wasser gewaschen. Nach dem Abdampfen des Lösungsmittels wird über 60 g basischem Aluminiumoxid (Alox B) mit CHCl₃ chromatographiert. Die günen Eluate werden vom Lösungsmittel befreit und der Rückstand aus Diethylether kristallisiert. Man erhält 1,4,8,11,15,18,22,25-Octakis-(2-methoxy-ethoxy)-bis-(diphenylcholesteryloxysiloxy)-germanium-phthalocyanin; Smp. 117-118°, λₘₐₓ (CHCl₃): 750nm.

Das Ausgangsmaterial erhält man folgendermassen:

Stufe 11.1: Eine Mischung aus 32 g (0,2 Mol) 2,3-Dicyano-hydrochinon, 104 g (0,24 Mol) 4-(2-Methoxy-ethyl)-toluolsulfonat, 126 g K₂CO₃ und 600 ml Dimethylformamid werden unter Stickstoff 2 Stunden bei 85° verrührt. Anschliessend wird im Vakuum zur Trockne verdampft, der Rückstand mit 400 ml Eiswasser und 400 ml CHCl₃ versetzt und filtriert. Der Filterrückstand wird gut mit CHCl₃ gewaschen. Nach Trocknen des Filterrückstandes erhält man 3,6-Bis-(2-methoxy-ethoxy)-phthalodinitril (analog J. Chem. Soc. Perk. Trans. 1988, 2453-58); Smp. 152-154°.

Sufe 11.2: Zu einer Lösung aus 1,1 g (0,16 Mol) Lithium in 120 ml Ethylenglykolmonomethylether werden 4,4 g (O,016 Mol) 3,6-Bis-(2-methoxy-ethoxy)-phthalodinitril (analog J. Chem. Soc. Perk. Trans. 1988, 2453-58) gegeben und 2,5 Stunden am Rückfluss erhitzt. Nach dem Abkühlen werden unter Eiskühlung 80 ml Essigsäure zugetropft. Man rührt eine Stunde bei Raumtemperatur, verdampft im Vakuum zur Trockne, löst den Rückstand in Methylenchlorid und wäscht mit einnormaler Salzsäure und Natriumchloridlösung. Nach dem Abdampfen des Lösungsmittels wird der Rückstand zweimal über Aluminiumoxid chromatographiert, zunächst in Tetrahydrofuran-Chloroform (1 : 1) und beim zweiten Mal in Tetrahydrofuran. Man erhält 1,4,8,11,15,18,22,25-Octakis-(2-methoxy-ethoxy)phthalocyanin; Smp. 156°, UV_{max.} = 760 nm (CHCl₃).

Stufe 11.3: Eine Mischung aus 1,1 g (1mMol) 1,4,8,11,15,18,22,25-Octakis-(2-methoxy-ethoxy)-phthalocyanin, 3,7 g (10 mMol) Tetrachlor-germanium-dimethylformamid-Komplex (hergestellt aus 7,3 g (0,1 Mol) Dimethylformamid, 2,1 g (0,01 Mol) Germaniumtetrachlorid in 50 ml Diethylether) und 60 ml Dimethylformamid wird 22 Stunden unter Rühren und einer Stickstoffatmosphäre am Rückfluss gekocht. Nach dem Abkühlen und Stehen während 24 Stunden bei Raumtemperatur wird 1,4,8,11,15,18,22,25-Octakis-(2-methoxy-ethoxy)-dichlorgermanium-phthalocyanin als schwarze, metallisch glänzende Kristalle erhalten; Smp. 272°C, UVₘₐₓ= 781 nm (CHCl₃).

Stufe 11.4: 1,6g (1,3 mmol) 1,4,8,11,15,18,22,25-Octakis-(2-methoxy-ethoxy)-dichlorgermanium-phthalocyanin werden mit 150 ml Chloroform und 100 ml 1N NaOH 3 Minuten bei Raumtemperatur geschüttelt. Anschliessend wird die wässrige Phase 1mal mit 50 ml gesättigter NaCl-Lösung gewaschen. Nach dem Abdampfen des Lösungsmittels wird 1,4,8,11,15,18,22,25-Octakis-(2-methoxy-ethoxy)-dihydroxy-germanium-phthalocyanin vom Smp. 214-216° erhalten; λₘₐₓ (CHCl₃): 762 nm.

Beispiel 12: Eine Mischung aus 121 mg (0,1 mMol) 1,4,8,11,15,18,22,25-Octakis-(2-methoxy-ethoxy)-dihydroxy-germanium-phthalocyanin (siehe Stufe 11.4), 319 mg (1 mMol) Chlortrihexylsilan, 500 mg Triethylamin und 20 ml β-Picolin wird 4 Stunden am Rückfluss erhitzt. Anschliessend wird im Vakuum zur Trockne verdampft, der Rückstand in Diethylether gelöst und 5mal mit gesättigter NaCl-Lösung gewaschen. Nach dem Trocknen über Natriumsulfat und Verdampfen des Lösungsmittels wird im Hochvakuum bei 80° 12 Stunden getrocknet. Auf diese Weise erhält man 1,4,8,11,15,18,22,25-Octakis-(2-methoxy-ethoxy)-bis-(tri-n-hexyl-silyloxy)-germanium-phthalocyanin; λₘₐₓ (CHCl₃): 756 nm.

Beispiel 13: Analog Beispiel 12 wird aus 1,4,8,11,15,18,22,25-Octakis(2-methoxy-ethoxy)-dihydroxy-germanium-phthalocyanin (siehe Stufe 11.4) und Texyldimethylchlorsilan 1,4,8,11,15,18,22,25-Octakis(2-methoxy-ethoxy)-bis(texyldimethylsiloxy)-germanium-phthalocyanin erhalten; λₘₐₓ (CHCl₃): 750 nm.

Beispiel 14: Eine Mischung aus 121 mg (0,1 mMol) 1,4,8,11,15,18,22,25-Octakis(2-methoxy-ethoxy)-dihydroxy-germanium-phthalocyanin (siehe Stufe 11.4), 2 mMol Triphenylsilanol, 3 ml Pyridin und 25 ml abs. Toluol wird eine Stunde am Rückfluss erhitzt. Anschliessend wird im Vakuum zur Trockne verdampft,der Rückstand in Diethylether gelöst und 4mal mit Wasser gewaschen. Nach dem Abdampfen des Lösungsmittels erhält man 1,4,8,11,15,18,22,25-Octakis(2-methoxy-ethoxy)-bis(triphenylsiloxy)-germanium-phthalocyanin; Smp. 149-151°, λₘₐₓ (CHCl₃): 759 nm.

Beispiel 15: 2,5 g Rohprodukt aus Stufe 15.1 und 1,32 g Dihydroxygermanium-phthalocyanin werden in 100 ml Dioxan während 50 Stunden unter Rückfluss erhitzt. Die erkaltete Lösung wird abgenutscht, das Filtrat zur Trockne eingedampft und der Rückstand mit Hexan versetzt. Die gewünschte Germaniumverbindung fällt als mikrokristallines Pulver aus. Dieses wird abgenutscht, gut mit Hexan gewaschen und getrocknet. Zur weiteren Reinigung wird aus CH₂Cl₂/Hexan umkristallisiert, worauf man Bis-(Diphenyl-[5α-cholestan-3β-yloxy]-siloxy)-germanium-phthalocyanin erhält; Smp. 279-280°, UV = 678 nm (CH₂Cl₂); NMR (CDCl₃): δ = unter anderem 9.54 (m) und 8.34 (m; je 8 H, Phthalocyanin); 6.7 (m), 6.28 (m) und 4.9 (m; total 20 H, Phenyl).

Stufe 15.1: Zu einer Lösung von 1 ml (4,9 mMol) Diphenylchlorsilan und 4,9 g Diisopropylaminomethylpolystyrol in 20 ml Toluol tropft man bei Raumtemperatur während 1 Stunde eine Lösung von 2 g (4,9 mMol) 5α-Cholestan-3β-ol in 25 ml Toluol. Man rührt das Reaktionsgemisch weitere 4 Stunden bei Raumtemperatur. Der sich gebildete Niederschlag wird abfiltriert, das Filtrat mit Essigsäureethylester verdünnt, je 2mal mit gesättigter Natriumhydrogencarbonatlösung und gesättigter Kochsalzlösung gewaschen und über Na₂SO₄ getrocknet. Das Lösungsmittel wird entfernt, der Rückstand mit Hexan versetzt. Der sich bildende Niederschlag wird abgenutscht, das Filtrat eingdampft und am Hochvakuum über Nacht getrocknet. Das erhaltene Rohprodukt wird ohne weitere Reinigung weiterverarbeitet; NMR(CDCl₃): u.a. m bei 7.68, 7.36 und 3.85.

Beispiel 16: 108 mg (0,175 mMol) Dihydroxygermanium-phthalocyanin und 154 mg (2 Äquivalente) Tetraacetylarbutin [hergestellt nach Arch. Pharm. 250, 547 ( 1912)] werden in 25 ml Toluol während 50 Stunden unter Rückfluss erhitzt. Die erkaltete Lösung wird abfiltriert, die Lösung zur Trockne eingdampft und der Rückstand aus CH₂Cl₂-Hexan umkristallisiert, worauf man Bis-[4-(2,3,4,6-tetra-O-acetyl-β-D-glucopyranos-1-yloxy)phenoxy]-germanium-phthalocyanin erhält; Smp. 250-252°, UV = 677 nm (CHCl₃); NMR(CDCl₃): δ = unter anderem 9.75 (m) und 8.55 (m; je 8 H, Phthalocyanin); 5.39 (d) und 2.67 (d; je 4 H, Phenyl); 2.12 (s), 2.08( 2mal s) und 1.98 (s, total 24 H, OAc).

Beispiel 17: Eine Mischung aus 121 mg (0,1 mMol) 1,4,8,11,15,18,22,25-Octakis(2-methoxy-ethoxy)-dihydroxy-germanium-phthalocyanin, 202 mg (1 mMol) n-1-Dodecanthiol, 3 ml Pyridin und 25 ml absolutem Toluol wird eine Stunde am Rückfluss erhitzt. Anschliessend wird im Vakuum zur Trockne verdampft und der Rückstand im Hochvakuum bei 75° 1,5 Stunden getrocknet. Auf diese Weise erhält man 1,4,8,11,15,18,22,25-Octakis(2-methoxy-ethoxy)-bis(n-1-dodecylthio)-germanium-phthalocyanin; λₘₐₓ (CHCl₃): 776 nm.

Beispiel 18: Analog Beispiel 17 wird aus 1,4,8,11,15,18,22,25-Octakis(2-methoxy-ethoxy)-dihydroxy-germanium-phthalocyanin und 1-Hexadecanol 1,4,8,11,15,18,22,25-Octakis(2-methoxy-ethoxy)-bis(n-hexadec-1-yloxy)-germanium-phthalocyanin erhalten; λₘₐₓ (CHCl₃): 761 nm.

Beispiel 19: Eine Mischung aus 121 mg (0,1 mMol) 1,4,8,11,15,18,22,25-Octakis-(2-methoxy-ethoxy)-dihydroxy-germanium-phthalocyanin, 148 mg (1 mMol) Diethylenglykol-monoacetat, 3 ml Pyridin und 25 ml absolutem Toluol wird 30 Minuten am Rückfluss erhitzt. Anschliessend wird zur Trockne verdampft, bei 75° im Hochvakuum getrocknet und aus Petrolether kristallisiert. Auf diese Weise erhält man 1,4,8,11,15,18,22,25-Octakis(2-methoxy-ethoxy)-bis(2-(2-acetoxy-ethoxy)-ethoxy)-germaniumphthalocyanin; Smp. 121-122°, λₘₐₓ(CHCl₃): 763 nm.

Beispiel 20: 200 mg 1,4,8,11,15,18,22,25-Octakis(2-methoxy-ethoxy)-dihydroxy-germanium-phthalocyanin werden in 10 ml Methanol 2 Minuten gekocht, filtriert, langsam abgekühlt und die ausgefallenen Kristalle abfiltriert. Man erhält 1,4,8,11,15,18,22,25-Octakis-(2-methoxy-ethoxy)-dimethoxy-germanium-phthalocyanin; Smp. 127-130°, λₘₐₓ (CHCl₃): 761 nm.

### Beispiel 21: Liposomenformulierung

Analog wie beschrieben in der Europäischen Patentanmeldung mit der Veröffentlichungsnummer 451103 erhält man eine Liposomenformulierung, enthaltend einen Gewichtsteil einer Verbindung der Formel I, 90 Gewichtsteile 1-n-Hexadecanoyl-2-(9-cis-octadecenoyl)-3-sn-phosphatidyl-cholin (POPC), 10 Gewichtsteile 1,2-Di-(9-cis-octadecenoyl)-3-sn-phosphatidyl-S-serin (OOPS) und 200 Gewichtsteile Lactose.

### Beispiel 22: Liposomenformulierung

Analog wie beschrieben in der Europäischen Patentanmeldung mit der Veröffentlichungsnummer 451103 erhält man eine Liposomenformulierung, enthaltend einen Gewichtsteil einer Verbindung der Formel I, 70 Gewichtsteile 1-n-Hexadecanoyl-2-(9-cis-octadecenoyl)-3-sn-phosphatidyl-cholin (POPC), 30 Gewichtsteile 1,2-Di-(9-cis-octadecenoyl)-3-sn-phosphatidyl-S-serin (OOPS) und 200 Gewichtsteile Lactose.

## Patentansprüche

1. Verbindungen der Formel I, 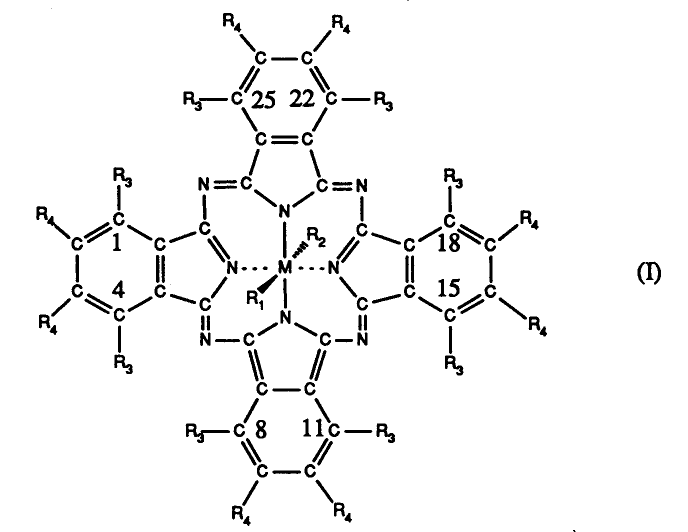 worin M ein Element der dritten oder vierten Hauptgruppe des Periodensystems mit einer Ordnungszahl von und mit 31 bis und mit 50, Aluminium oder Ruthenium bedeutet, R₁ für einen Rest 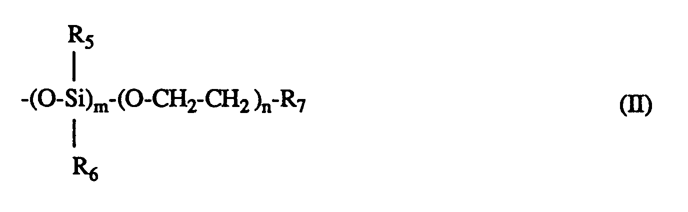 der Formel II, worin m für 0 oder 1 steht, n eine ganze Zahl von und mit 0 bis und mit 200, R₅ und R₆ unabhängig voneinander je Niederalkyl oder gegebenenfalls substituiertes Phenyl und R₇ Alkylthio mit bis zu 20 C-Atomen, Alkanoyloxy mit bis zu 20 C-Atomen, 4-(2,3,4,6-Tetra-O-acetyl-β-D-glucopyranos-1-yloxy)-phenoxy, Cholestan-3-yl-oxy, Cholesteryloxy, unsubstituiertes oder durch Cholestan-3-yl-oxy oder Cholesteryloxy substituiertes aliphatisches Hydrocarbyloxy mit bis zu 24 C-Atomen im aliphatischen Teil oder, wenn m für 1 und n für 0 steht, aliphatisches Hydrocarbyl mit 6-24 C-Atomen oder unsubstituiertes oder substituiertes Phenyl bedeuten, oder R₁ für einen Rest der Formel III steht, 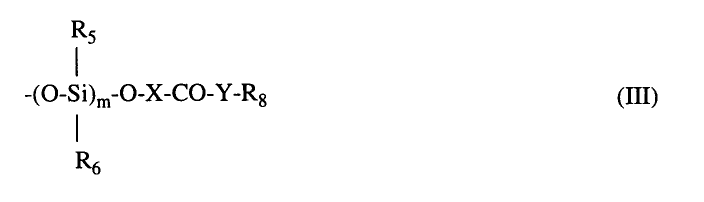 worin X einen bivalenten aliphatischen Kohlenwasserstoffrest mit bis zu 23 C-Atomen bedeutet, Y für Sauerstoff oder die Gruppe -NH- steht, R₈ für Cholesteryl, aliphatisches Hydrocarbyl mit bis zu 24 C-Atomen oder unsubstituiertes oder durch Cholesteryloxy substituiertes aliphatisches Hydrocarbyl mit bis zu 24 C-Atomen im aliphatischen Teil steht, und R₅, R₆ und m die obengenannten Bedeutungen haben, R₂ die Bedeutung von R₁ hat, wenn M ein Element der vierten Hauptgruppe ist, oder R₂ fehlt, wenn M ein Element der dritten Hauptgruppe oder Ruthenium ist, R₃ Wasserstoff, Niederalkyl, Niederalkylthio, unsubstituiertes oder durch Niederalkoxy substituiertes Alkoxy mit bis zu 20 C-Atomen, Triniederalkyl-silyl oder Halogen bedeutet und R₄ für Wasserstoff, Niederalkyl, Niederalkylthio, unsubstituiertes oder durch Niederalkoxy substituiertes Alkoxy mit bis zu 20 C-Atomen, Triniederalkyl-silyl oder Halogen steht, mit Ausnahme der Verbindung der Formel I, worin M für Zinn, R₁ und R₂ je für den Rest -OSi(C₆H₁₃)₃ und R₃ und R₄ je für tert. Butyl stehen.

2. Verbindungen nach Anspruch 1 der Formel I, worin R₁ für einen Rest der Formel II steht, worin R₇ Cholesteryloxy, unsubstituiertes oder durch Cholesteryloxy substituiertes aliphatisches Hydrocarbyloxy mit bis zu 24 C-Atomen im aliphatischen Teil oder, wenn m für 1 und n für 0 steht, aliphatisches Hydrocarbyl mit 6-24 C-Atomen bedeutet, oder worin R₁ für einen Rest der Formel III steht, worin X einen bivalenten aliphatischen Kohlenwasserstoffrest mit bis zu 23 C-Atomen bedeutet, Y für Sauerstoff oder die Gruppe -NH-steht, und R₈ für Cholesteryl, aliphatisches Hydrocarbyl mit bis zu 24 C-Atomen oder unsubstituiertes oder durch Cholesteryloxy substituiertes aliphatisches Hydrocarbyl mit bis zu 24 C-Atomen im aliphatischen Teil steht, mit Ausnahme der Verbindung der Formel I, worin M für Zinn, R₁ und R₂ je für den Rest -OSi(C₆H₁₃)₃ und R₃ und R₄ je für tert. Butyl stehen.

3. Verbindungen der Formel I nach Anspruch 1, worin M für Germanium steht, R₁ und R₂ je einen und denselben Rest der Formel II, worin m für 0 oder 1 steht, n für eine ganze Zahl von und mit 0 bis und mit 20 steht, R₅ und R₆ unabhängig voneinander je für Niederalkyl oder Phenyl stehen und R₇ für Cholesteryloxy, Alkyl mit 6-24 C-Atomen, Alkoxy mit bis zu 24 C-Atomen, Alkenyloxy mit bis zu 24 C-Atomen oder für ω-Cholesteryloxy-alkoxy mit bis zu 24 C-Atomen im Alkoxyteil steht, oder worin R₁ und R₂ je einen und denselben Rest der Formel III, worin m für 0 oder 1 steht, X für Alkylen mit bis zu 23 C-Atomen steht, Y für Sauerstoff steht, R₅ und R₆ je für Phenyl oder Niederalkyl stehen und R₈ für Cholesteryl steht, bedeuten, R₃ für Wasserstoff, Triniederalkylsilyl oder für unsubstituiertes oder durch Niederalkoxy substituiertes Alkoxy mit bis zu 20 C-Atomen steht, und R₄ Wasserstoff bedeutet.

4. Verbindungen der Formel I nach Anspruch 1, worin M für Germanium steht, R₁ und R₂ je einen und denselben Rest der Formel II, worin m für 0 oder 1 steht, n für eine ganze Zahl von und mit 0 bis und mit 5 steht, R₅ und R₆ je für Phenyl oder Niederalkyl stehen und R₇ für Cholesteryloxy, Alkoxy mit bis zu 18 C-Atomen, C₁₈-Alkenyloxy, Alkyl mit 6 bis 18 C-Atomen, ω-Cholesteryloxy-C₁₂₋₁₈-alkoxy, Phenyl, Cholestan-3-yloxy, 4-(2,3,4,6-Tetra-O-acetyl-β-D-glucopyranos-1-yloxy)-phenoxy, C₁₀₋₁₄-Alkylthio oder Niederalkanoyloxy steht, oder worin R₁ und R₂ je einen und denselben Rest der Formel III bedeuten, worin m für 1, X für C₁₁₋₁₇-Alkylen, Y für Sauerstoff, R₅ und R₆ je für Phenyl und R₈ für Cholesteryl stehen, R₃ Wasserstoff oder durch Niederalkoxy substituiertes Niederalkoxy und R₄ Wasserstoff bedeuten.

5. Verbindungen der Formel I nach Anspruch 1, worin M für Germanium steht, R₁ und R₂ je einen und denselben Rest der Formel II, worin m für 0 oder 1 steht, n für 0, 2, 3 oder 5 steht, R₅ und R₆ je für Phenyl, Methyl oder n-Hexyl stehen und R₇ für Cholesteryloxy, Methoxy, n-Hexadecyloxy, cis-9-Octadecenyloxy, n-Hexyl, 1,1,2-Trimethyl-propyl, n-Octadecyl, ω-Cholesteryloxy-n-hexadecyloxy, Phenyl, 5-α-Cholestan-3-β-yloxy, 4-(2,3,4,6-Tetra-O-acetyl-β-glucopyranos-1-yloxy)-phenoxy, n-Dodecylthio oder Acetoxy steht, oder worin R₁ und R₂ je einen und denselben Rest der Formel III bedeuten, worin m für 1, X für n-Pentadecylen, Y für Sauerstoff, R₅ und R₆ je für Phenyl und R₈ für Cholesteryl stehen, R₃ Wasserstoff oder 2-Methoxy-ethoxy und R₄ Wasserstoff bedeuten.

6. Bis-(Diphenylcholesteryloxysiloxy)-germanium-phthalocyanin nach Anspruch 1.

7. Eine Verbindung nach Anspruch 1 der Formel I, ausgewählt aus Bis-(Diphenylhexadecyloxy-siloxy)-germanium-phthalocyanin, B is-(Diphenyl-cis-9-octadecenyloxy-siloxy)-germanium-phthalocyanin, Bis-(Diphenyl-octadecyloxy-siloxy)-germanium-phthalocyanin, Bis-(Dimethyl-octadecyl-siloxy)-germanium-phthalocyanin, Bis-[(5-cholesteryloxy-3-oxa-pentyloxy)-diphenyl-siloxy]-germanium-phthalocyanin, Bis-[Diphenyl-(3,6,9,12,15-pentaoxa-hexadec-1-yloxy)-siloxy]-germanium-phthalocyanin, Bis-[Diphenyl-(ω-cholesteryloxycarbonyl-n-pentadec-1-yloxy)-siloxy]-germanium-phthalocyanin, 1,4,8,11,15,18,22,25-Octakis-(2-methoxy-ethoxy)-bis-(diphenylcholesteryloxysiloxy)-germanium-phthalocyanin, 1,4,8,11,15,18,22,25-Octakis(2-methoxy-ethoxy)-bis-(tri-n-hexyl-silyloxy)-germanium-phthalocyanin, 1,4,8,11,15,18,22,25-Octakis(2-methoxy-ethoxy)-bis(texyldimethylsiloxy)-germanium-phthalocyanin, 1,4,8,11,15,18,22,25-Octakis(2-methoxy-ethoxy)-bis(triphenylsiloxy)-germanium-phthalocyanin, Bis-(Diphenyl-[5α-cholestan-3β-yloxy]-siloxy)-germanium-phthalocyanin, Bis-[4-(2,3,4,6-tetra-O-acetyl-β-D-glucopyranos-1-yloxy)-phenoxy]-germanium-phthalocyanin, 1,4,8,11,15,18,22,25-Octakis(2-methoxy-ethoxy)-bis(n-1-dodecylthio)-germanium-phthalocyanin, 1,4,8,11,15,18,22,25-Octakis(2-methoxy-ethoxy)-bis(n-hexadec-1-yloxy)-germanium-phthalocyanin, 1,4,8,11,15,18,22,25-Octakis(2-methoxy-ethoxy)-bis(2-(2-acetoxy-ethoxy)-ethoxy)-germanium-phthalocyanin und 1,4,8,11,15,18,22,25-Octakis(2-methoxy-ethoxy)-dimethoxy-germanium-phthalocyanin.

8. Pharmazeutische Zusammensetzung, enthaltend eine Verbindung der Formel I gemäss einem der Ansprüche 1-7 zusammen mit pharmazeutischem Trägermaterial.

9. Eine Verbindung der Formel I gemäss einem der Ansprüche 1-7 zur Anwendung in einem Verfahren zur therapeutischen Behandlung des menschlichen oder tierischen Körpers.

10. Verwendung einer Verbindung der Formel I gemäss einem der Ansprüche 1-7 zur Herstellung von pharmazeutischen Zusammensetzungen zur Anwendung zur photodynamischen Chemotherapie von Tumoren.

11. Verfahren zur Herstellung einer Verbindung der Formel I gemäss Anspruch 1, dadurch gekennzeichnet, dass man eine Verbindung der Formel V, 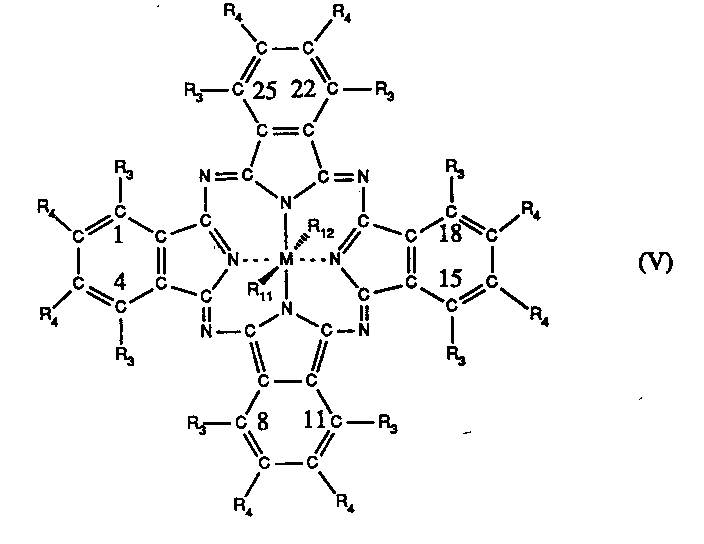 worin R₁₁ für Hydroxy oder Halogen steht und R₁₂ die Bedeutung von R₁₁ hat, wenn M ein Element der vierten Hauptgruppe ist, oder R₁₂ fehlt, wenn M ein Element der dritten Hauptgruppe oder Ruthenium ist, und die übrigen Symbole die obengenannten Bedeutungen haben, mit einer Verbindung der Formel VI,
R₁-H (VI)
worin R₁ die obengenannten Bedeutungen hat, oder mit einem reaktionsfähigen Derivat davon mit aktivierter OH-Gruppe umsetzt.

## Claims

1. A compound of formula I 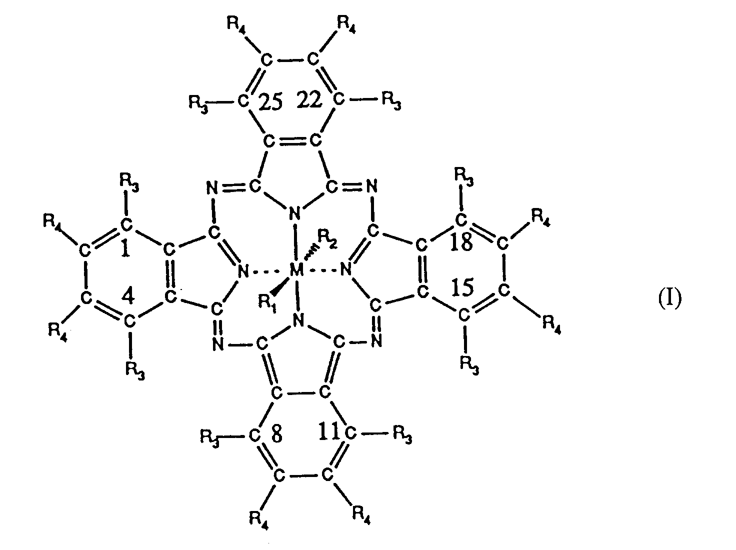 wherein
M is an element of main group III or IV of the Periodic Table having an atomic number of from 31 to 50 inclusive, aluminium or ruthenium,
R₁ is a radical of formula II 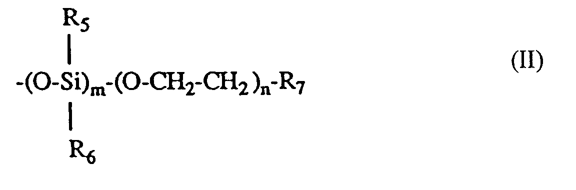 wherein
m is 0 or 1,
n is an integer from 0 to 200 inclusive,
R₅ and R₆ are each independently of the other lower alkyl or unsubstituted or substituted phenyl, and
R₇ is alkylthio having up to 20 carbon atoms, alkanoyloxy having up to 20 carbon atoms, 4-(2,3,4,6-tetra-O-acetyl-β-D-glucopyranos-1-yloxy)-phenoxy, cholestan-3-yloxy, cholesteryloxy, unsubstituted or cholestan-3-yloxy- or cholesteryloxy-substituted aliphatic hydrocarbyloxy having up to 24 carbon atoms in the aliphatic moiety or, when m is 1 and n is 0, aliphatic hydrocarbyl having from 6 to 24 carbon atoms, or unsubstituted or substituted phenyl, or R₁ is a radical of formula III 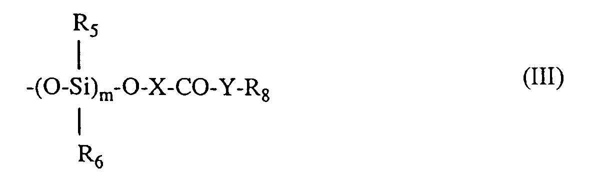 wherein
X is a bivalent aliphatic hydrocarbon radical having up to 23 carbon atoms,
Y is oxygen or the group -NH-,
R₈ is cholesteryl, aliphatic hydrocarbyl having up to 24 carbon atoms or unsubstituted or cholesteryloxy-substituted aliphatic hydrocarbyl having up to 24 carbon atoms in the aliphatic moiety, and
R₅, R₆ and m are as defined above,
R₂ has the same definition as R₁ when M is an element of main group IV, or R₂ is absent when M is an element of main group III or ruthenium,
R₃ is hydrogen, lower alkyl, lower alkylthio, unsubstituted or lower alkoxy-substituted alkoxy having up to 20 carbon atoms, tri-lower alkylsilyl or halogen, and
R₄ is hydrogen, lower alkyl, lower alkylthio, unsubstituted or lower alkoxy-substituted alkoxy having up to 20 carbon atoms, tri-lower alkylsilyl or halogen,
with the exception of the compound of formula I wherein M is tin, R₁ and R₂ are each the radical ―OSi(C₆H₁₃)₃ and R₃ and R₄ are each tert-butyl.

2. A compound according to claim 1 of formula I, wherein
R₁ is a radical of formula II wherein R₇ is cholesteryloxy, unsubstituted or cholesteryloxy-substituted aliphatic hydrocarbyloxy having up to 24 carbon atoms in the aliphatic moiety or, when m is 1 and n is 0, aliphatic hydrocarbyl having from 6 to 24 carbon atoms, or wherein
R₁ is a radical of formula III wherein X is a bivalent aliphatic hydrocarbon radical having up to 23 carbon atoms, Y is oxygen or the group -NH-, and R₈ is cholesteryl, aliphatic hydrocarbyl having up to 24 carbon atoms or unsubstituted or cholesteryloxy-substituted aliphatic hydrocarbyl having up to 24 carbon atoms in the aliphatic moiety,
with the exception of the compound of formula I wherein M is tin, R₁ and R₂ are each the radical ―OSi(C₆H₁₃)₃ and R₃ and R₄ are each tert-butyl.

3. A compound of formula I according to claim 1, wherein
M is germanium,
R₁ and R₂ each represents the same radical of formula II wherein m is 0 or 1, n is an integer from 0 to 20 inclusive, R₅ and R₆ are each independently of the other lower alkyl or phenyl and R₇ is cholesteryloxy, alkyl having from 6 to 24 carbon atoms, alkoxy having up to 24 carbon atoms, alkenyloxy having up to 24 carbon atoms or ω-cholesteryloxy-alkoxy having up to 24 carbon atoms in the alkoxy moiety, or wherein
R₁ and R₂ each represents the same radical of formula III wherein m is 0 or 1, X is alkylene having up to 23 carbon atoms, Y is oxygen, R₅ and R₆ are each phenyl or lower alkyl and R₈ is cholesteryl,
R₃ is hydrogen, tri-lower alkylsilyl or unsubstituted or lower alkoxy-substituted alkoxy having up to 20 carbon atoms, and
R₄ is hydrogen.

4. A compound of formula I according to claim 1, wherein
M is germanium,
R₁ and R₂ each represents the same radical of formula II wherein m is 0 or 1, n is an integer from 0 to 5 inclusive, R₅ and R₆ are each phenyl or lower alkyl and R₇ is cholesteryloxy, alkoxy having up to 18 carbon atoms, C₁₈alkenyloxy, alkyl having from 6 to 18 carbon atoms, ω-cholesteryloxy-C₁₂₋₁₈alkoxy, phenyl, cholestan-3-yl-oxy, 4-(2,3,4,6-tetra-O-acetyl-β-D-glucopyranos-1-yloxy)-phenoxy, C₁₀₋₁₄alkylthio or lower alkanoyloxy, or wherein
R₁ and R₂ each represents the same radical of formula III wherein m is 1, X is C₁₁₋₁₇alkylene, Y is oxygen, R₅ and R₆ are each phenyl and R₈ is cholesteryl,
R₃ is hydrogen or lower alkoxy-substituted lower alkoxy, and
R₄ is hydrogen.

5. A compound of formula I according to claim 1, wherein
M is germanium,
R₁ and R₂ each represents the same radical of formula II wherein m is 0 or 1, n is 0, 2, 3 or 5, R₅ and R₆ are each phenyl, methyl or n-hexyl and R₇ is cholesteryloxy, methoxy, n-hexadecyloxy, cis-9-octadecenyloxy, n-hexyl, 1,1,2-trimethyl-propyl, n-octadecyl, ω-cholesteryloxy-n-hexadecyloxy, phenyl, 5-α-cholestan-3-β-yloxy, 4-(2,3,4,6-tetra-O-acetyl-β-D-glucopyranos-1-yloxy)-phenoxy, n-dodecylthio or acetoxy, or wherein
R₁ and R₂ each represents the same radical of formula III wherein m is 1, X is n-penta-decylene, Y is oxygen, R₅ and R₆ are each phenyl and R₈ is cholesteryl,
R₃ is hydrogen or 2-methoxy-ethoxy, and
R₄ is hydrogen.

6. Bis(diphenylcholesteryloxysiloxy)germanium-phthalocyanine according to claim 1.

7. A compound according to claim 1 of formula I, selected from bis(diphenylhexadecyloxy-siloxy)germanium-phthalocyanine, bis(diphenyl-cis-9-octadecenyloxy-siloxy)germanium-phthalocyanine, bis(diphenyl-octadecyloxy-siloxy)germanium-phthalocyanine, bis(dimethyl-octadecyl-siloxy)germanium-phthalocyanine, bis [(5-cholesteryloxy-3-oxapentyloxy)-diphenyl-siloxy]germanium-phthalocyanine, bis[diphenyl-(3,6,9,12,15-pentaoxa-hexadec-1-yloxy)-siloxy]germanium-phthalocyanine, bis[diphenyl-(ω-cholesteryloxycarbonyl-n-pentadec-1-yloxy)-siloxy]germanium-phthalocyanine, 1,4,8,11,15,18,22,25-octakis(2-methoxy-ethoxy)-bis(diphenylcholesteryloxysiloxy)germanium-phthalocyanine, 1,4,8,11,15,18,22,25-octakis(2-methoxy-ethoxy)-bis(tri-n-hexyl-silyloxy)germanium-phthalocyanine, 1,4,8,11,15,18,22,25-octakis(2-methoxy-ethoxy)-bis(texyldimethylsiloxy)germanium-phthalocyanine, 1,4,8,11,15,18,22,25-octakis(2-methoxy-ethoxy)-bis(triphenylsiloxy)germanium-phthalocyanine, bis(diphenyl-[5α-cholestan-3β-yloxy]-siloxy)germanium-phthalocyanine, bis[4-(2,3,4,6-tetra-O-acetyl-β-D-glucopyranos-1-yloxy)-phenoxy] germanium-phthalocyanine, 1,4,8,11,15,18,22,25-octakis(2-methoxy-ethoxy)-bis(n-1-dodecylthio)germanium-phthalocyanine, 1,4,8,11,15,18,22,25-octakis(2-methoxy-ethoxy)-bis(n-hexadec-1-yloxy)germanium-phthalocyanine, 1,4,8,11,15,18,22,25-octakis(2-methoxy-ethoxy)-bis(2-(2-acetoxy-ethoxy)-ethoxy)germanium-phthalocyanine and 1,4,8,11,15,18,22,25-octakis(2-methoxy-ethoxy)dimethoxy-germanium-phthalocyanine.

8. A pharmaceutical composition comprising a compound of formula I according to any one of claims 1 to 7 together with a pharmaceutical carrier.

9. A compound of formula I according to any one of claims 1 to 7 for use in a method for the therapeutic treatment of the human or animal body.

10. The use of a compound of formula I according to any one of claims 1 to 7 for the preparation of pharmaceutical compositions for use in the photodynamic chemotherapy of tumours.

11. A process for the preparation of a compound of formula I according to claim 1, wherein a compound of formula V 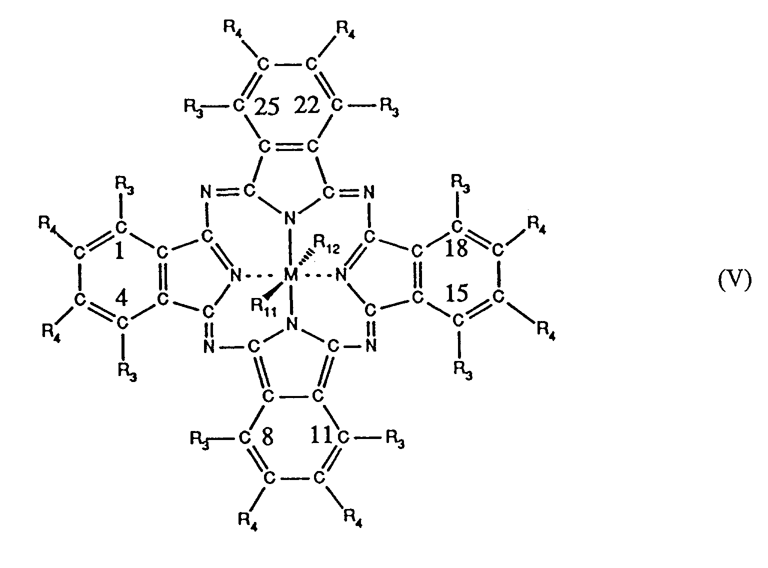 wherein
R₁₁ is hydroxy or halogen, and
R₁₂ has the same definition as R₁₁ when M is an element of main group IV, or R₁₂ is absent when M is an element of main group III or ruthenium,
and the other symbols are as defined above, is reacted with a compound of formula VI
R₁-H (VI)
wherein R₁ is as defined above, or with a reactive derivative thereof having an activated OH group.

## Revendications

1. L 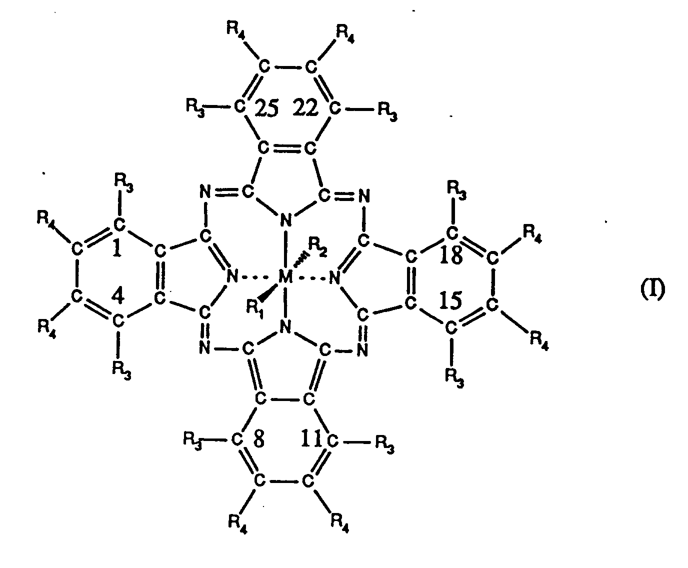 es composés de formule I, où M signifie un élément du 3ème ou du 4ème groupe principal du système périodique avec un nombre atomique de 31 à 50, l'aluminium ou le ruthénium, R₁ signifie un reste de formule II, 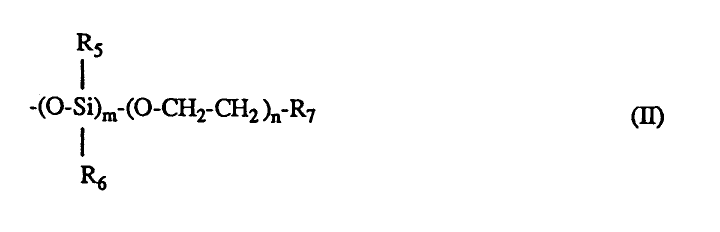 où m signifie 0 ou 1, n signifie un nombre entier de 0 à 200, R₅ et R₆ indépendamment l'un de l'autre, signifient chacun un groupe alkyle inférieur ou un groupe phényle éventuellement substitué et R₇ signifie un groupe alkylthio ayant jusqu'à 20 atomes de carbone, un groupe alcanoyloxy ayant jusqu'à 20 atomes de carbone, un groupe 4-(2,3,4,6-tétra-O-acétyl-β-D-glucopyranos-1-yloxy)-phénoxy,cholestan-3-yl-oxy, cholestéryloxy, hydrocarbyloxy aliphatique non substitué ou substitué par un groupe cholestan-3-yl-oxy ou par un groupe cholestéryloxy, ayant jusqu'à 24 atomes de carbone dans la partie aliphatique ou bien, lorsque m signifie 1 et n signifie 0, un groupe hydrocarbyle aliphatique ayant 6-24 atomes de carbone ou un groupe phényle non substitué ou substitué, ou bien R₁ signifie un reste de formule III, 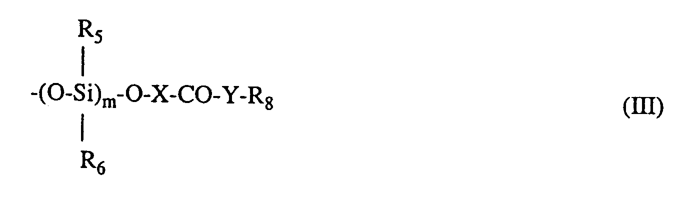 où X signifie un reste hydrocarboné aliphatique bivalent ayant jusqu'à 23 atomes de carbone, Y signifie l'oxygène ou le groupe -NH-, R₈ signifie un groupe cholestéryle, hydrocarbyle aliphatique ayant jusqu'à 24 atomes de carbone ou bien un groupe hydrocarbyle aliphatique non substitué ou substitué par un groupe cholestéryloxy, ayant jusqu'à 24 atomes de carbone dans la partie aliphatique, et R₅, R₆ et m ont les significations susmentionnées, R₂ a la signification de R₁, lorsque M signifie un élément du 4ème groupe principal, ou bien R₂ est absent lorsque M signifie un élément du 3ème groupe principal ou le ruthénium, R₃ signifie l'hydrogène, un groupe alkyle inférieur, alkylthio inférieur, un groupe alcoxy non substitué ou substitué par un groupe alcoxy inférieur, ayant jusqu'à 20 atomes de carbone, un groupe Tri-alkyl inférieur-silyle ou un halogène et R₄ signifie l'hydrogène, un groupe alkyle inférieur, alkylthio inférieur, alcoxy non substitué ou substitué par un groupe alcoxy inférieur, ayant jusqu'à 20 atomes de carbone, un groupe Tri-alkyl inférieur-silyle ou un halogène, à l'exception du composé de formule I, où M signifie l'étain, R₁ et R₂ signifient chacun un reste -OSi(C₆H₁₃)₃ et R₃ et R₄ signifient chacun un groupe tert.-butyle.

2. Les composés selon la revendication 1 de formule I, où R₁ signifie un reste de formule II, où R₇ signifie un groupe cholestéryloxy, hydrocarbyloxy aliphatique non substitué ou substitué par un groupe cholestéryloxy, ayant jusqu'à 24 atomes de carbone dans la partie aliphatique, ou bien, lorsque m signifie 1 et n signifie 0, un groupe hydrocarbyle aliphatique ayant 6-24 atomes de carbone, ou bien où R₁ signifie un reste de formule III, où X signifie un reste hydrocarboné aliphatique bivalent ayant jusqu'à 23 atomes de carbone, Y signifie l'oxygène ou le groupe -NH- et R₈ signifie un groupe cholestéryle, un groupe hydrocarbyle aliphatique ayant jusqu'à 24 atomes de carbone ou bien un groupe hydrocarbyle aliphatique non substitué ou substitué par un groupe cholestéryloxy, ayant jusqu'à 24 atomes de carbone dans la partie aliphatique, à l'exception du composé de formule I, où M signifie l'étain, R₁ et R₂ signifient chacun un reste -OSi(C₆H₁₃)₃ et R₃ et R₄ signifient chacun un groupe tert.-butyle.

3. Les composés de formule I selon la revendication 1, où M signifie le germanium, R₁ et R₂ signifient chacun un reste et le même reste de formule II, où m signifie 0 ou 1, n signifie un nombre entier de 0 à 20, R₅ et R₆ indépendamment l'un de l'autre, signifient chacun un groupe alkyle inférieur ou un groupe phényle et R₇ signifie un groupe cholestéryloxy, alkyle ayant 6-24 atomes de carbone, alcoxy ayant jusqu'à 24 atomes de carbone, alcényloxy ayant jusqu'à 24 atomes de carbone ou un groupe ω-cholestéryloxy-alcoxy ayant jusqu'à 24 atomes de carbone dans la partie alcoxy, ou bien où R₁ et R₂ signifient chacun un reste et le même reste de formule III, où m signifie 0 ou 1, X signifie un groupe alkylène ayant jusqu'à 23 atomes de carbone, Y signifie l'oxygène, R₅ et R₆ signifient chacun un groupe phényle ou alkyle inférieur et R₈ signifie un groupe cholestéryle, R₃ signifie l'hydrogène, un groupe Tri-alkyl inférieur-silyle ou bien un groupe alcoxy non substitué ou substitué par un groupe alcoxy inférieur, ayant jusqu'à 20 atomes de carbone, et R₄ signifie l'hydrogène.

4. Les composés de formule I selon la revendication 1, où M signifie le germanium, R₁ et R₂ signifient chacun un reste et le même reste de formule II, où m signifie 0 ou bien 1, n signifie un nombre entier de 0 à 5, R₅ et R₆ signifient chacun un groupe phényle ou alkyle inférieur et R₇ signifie un groupe cholestéryloxy, alcoxy ayant jusqu'à 18 atomes de carbone, C₁₈-Alcényloxy, alkyle ayant de 6 à 18 atomes de carbone, ω-cholestéryloxy-C₁₂₋₁₈-alcoxy, phényle, cholestan-3-yloxy, 4-(2,3,4,6-tétra-O-acétyl-β-D-glucopyranos-1-yloxy)-phénoxy, C₁₀₋₁₄-alkylthio ou bien alcanoyloxy inférieur, ou bien où R₁ et R₂ signifient chacun un reste et le même reste de formule III, où m signifie 1, X signifie un groupe C₁₁₋₁₇alkylène, Y signifie l'oxygène, R₅ et R₆ signifient chacun un groupe phényle et R₈ signifie un groupe cholestéryle, R₃ signifie l'hydrogène ou un groupe alcoxy inférieur substitué par un groupe alcoxy inférieur et R₄ signifie l'hydrogène.

5. Les composés de formule I selon la revendication 1, où M signifie le germanium, R₁ et R₂ signifient chacun un reste et le même reste de formule II, où m signifie 0 ou bien 1, n signifie 0, 2, 3 ou 5, R₅ et R₆ signifient chacun un groupe phényle, méthyle ou n-hexyle et R₇ signifie un groupe cholestéryloxy, méthoxy, n-hexadécyloxy, cis-9-octadécényloxy, n-hexyle, 1,1,2-triméthyl-propyle, n-octadécyle, ω-cholesteryloxy-n-hexadécyloxy, phényle, 5-α-cholestan-3-β-yloxy, 4-(2,3,4,6-tétra-O-acétyl-β-D-glucopyranos-1-yloxy)-phénoxy, n-dodécylthio ou acétoxy, ou bien où R₁ et R₂ signifient chacun un reste et le même reste de formule III, où m signifie 1, X signifie un groupe n-pentadécylène, Y signifie l'oxygène, R₅ et R₆ signifient chacun un groupe phényle et R₈ signifie un groupe cholestéryle, R₃ signifie l'hydrogène ou un groupe 2-méthoxy-éthoxy et R₄ signifie l'hydrogène.

6. La bis-(Diphénylcholestéryloxysiloxy)-germanium-phtalocyanine selon la revendication 1.

7. Un composé selon la revendication 1 de formule I, choisi parmi la bis(diphénylhexadécyloxy-siloxy)-germanium-phtalocyanine, la bis-(diphényl-cis-9-octadécenyloxy-siloxy)-germanium-phtalocyanine, la bis-(diphényl-octadécyloxysiloxy)-germanium-phtalocyanine, la bis-(diméthyl-octadécyl-siloxy)-germanium-phtalocyanine, la bis-[(5-cholestéryloxy-3-oxa-pentyloxy)-diphényl-siloxy]-germanium-phtalocyanine, la bis-[diphényl-(3,6,9,12,15-pentaoxa-hexadec-1-yloxy)-siloxy]-germanium-phtalocyanine, la bis-[diphényl-(ω-cholestéryloxycarbonyl-n-pentadec-1-yloxy)-siloxy]-germanium-phtalocyanine, la 1,4,8,11,15,18,22,25-octakis-(2-méthoxy-éthoxy)-bis-(diphénylcholestéryloxysiloxy)-germanium-phtalocyanine, la 1,4,8,11,15,18,22,25-octakis(2-méthoxy-éthoxy)-bis-(tri-n-hexyl-silyloxy)-germanium-phtalocyanine, la 1,4,8,11,15,18,22,25-octakis(2-méthoxy-éthoxy)-bis(texyldiméthylsiloxy)-germanium-phtalocyanine, la 1,4,8,11,15,18,22,25-octakis(2-méthoxy-éthoxy)-bis(triphénylsiloxy)-germanium-phtalocyanine, la bis-(diphényl-[5α-cholestan-3β-yloxy]-siloxy)-germanium-phtalocyanine, la bis-[4-(2,3,4,6-tétra-O-acétyl-β-D-glucopyranos-1-yloxy)-phénoxy]-germanium-phtalocyanine, la 1,4,8,11,15,18,22,25-octakis(2-méthoxy-éthoxy)-bis(n-1-dodécylthio)-germanium-phtalocyanine, la 1,4,8,11,15,18,22,25-octakis(2-méthoxy-éthoxy)-bis(n-hexadec-1-yloxy)-germanium-phtalocyanine, la 1,4,8,11,15,18,22,25-octakis(2-méthoxy-éthoxy)-bis(2-(2-acétoxy-éthoxy)-éthoxy)-germanium-phtalocyanine, la 1,4,8,11,15,18,22,25-octakis(2-méthoxy-éthoxy)-diméthoxy-germanium-phtalocyanine.

8. Une composition pharmaceutique contenant un composé de formule I selon l'une quelconque des revendications 1-7, ensemble avec un véhicule pharmaceutique.

9. Un composé de formule I selon l'une quelconque des revendications 1-7 pour une utilisation dans un procédé de traitement thérapeutique du corps humain ou animal.

10. Utilisation d'un composé de formule I selon l'une quelconque des revendications 1-7 pour la préparation de compositions pharmaceutiques pour l'utilisation pour la chimiothérapie photodynamique de tumeurs.

11. Un procédé de préparation d'un composé de formule I selon la revendication 1, caractérisé en ce qu'on fait réagir un composé de formule V, 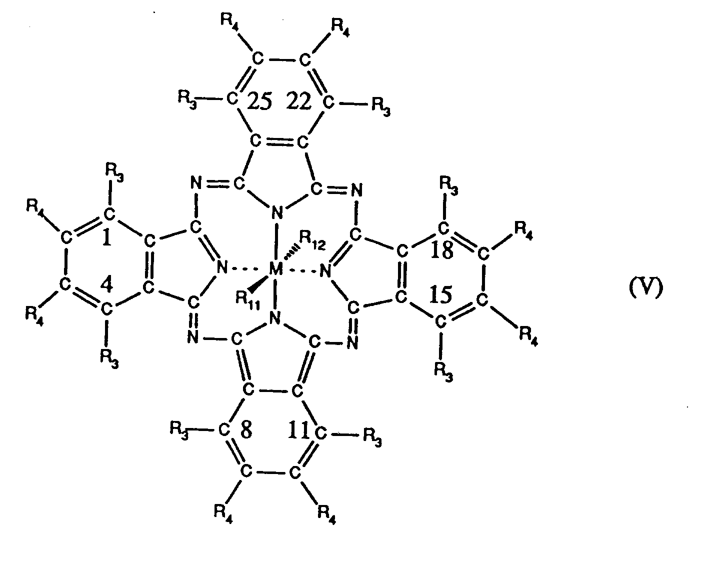 où R₁₁ signifie un groupe hydroxy ou un halogène et R₁₂ a la signification de R₁₁, lorsque M signifie un élément du 4ème groupe principal, ou bien R₁₂ est absent, lorsque M signifie un élément du 3ème groupe principal ou le ruthénium, et les autres symboles ont les significations susmentionnées, avec un composé de formule VI,
R₁-H (VI)
où R₁ a les significations susmentionnées, ou bien avec un de ses dérivés réactifs ayant un groupe OH activé.
